# FASCICULE DE BREVET EUROPEEN

(11) **EP 2 069 342 B1**
(45) Date de publication et mention de la délivrance du brevet: **12.05.2010**
(21) Numéro de dépôt: 07848251.0
(22) Date de dépôt: 19.09.2007
(51) Int. Cl.: C07D 471/04, A61K 31/437, A61P 25/00, A61P 19/10, A61P 35/00

(54) **DERIVES DE 2-ARYL-6-PHENYL-IMIDAZO[1,2-A]PYRIDINES, LEUR PREPARATION ET LEUR APPLICATION EN THERAPEUTIQUE**
2-ARYL-6-PHENYLIMIDAZO[1,2-A]PYRIDIN-DERIVATE, IHRE HERSTELLUNG UND THERAPEUTISCHE VERWENDUNG
2-ARYL-6-PHENYLIMIDAZO[1,2-A]PYRIDINE DERIVATIVES, PREPARATION THEREOF AND THERAPEUTIC USE THEREOF

(30) Priorité: 22.09.2006 FR 0608350
(43) Date de publication de la demande: 17.06.2009
(73) Titulaire: Sanofi-Aventis, 75013 Paris (FR)
(72) Inventeur: ALMARIO GARCIA, Antonio, F-75013 Paris (FR); LARDENOIS, Patrick, F-75013 Paris (FR); OLIVIER, Anne, F-75013 Paris (FR)
(74) Mandataire: Morel-Pécheux, Muriel
(86) Numéro de dépôt international: PCT/FR2007/001517
(87) Numéro de publication internationale: WO 2008/034974

(56) Documents cités:
- WO-A-01/74813
- WO-A-2004/026867
- WO-A-2004/072050
- WO-A-2005/086808

## Description

La présente invention se rapporte à des dérivés de 2-aryl-6-phényl-imidazo[1,2-*a*]pyridine, à leur préparation et à leur application en thérapeutique dans le traitement ou la prévention de maladies impliquant les récepteurs nucléaires Nurr-1 aussi appelé NR4A2, NOT, TINUR, RNR-1, et HZF3.

La présente invention a pour objet les composés répondant à la formule (I) : 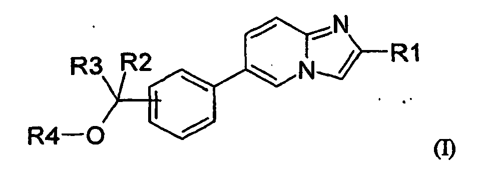 dans laquelle :
- R₁ représente :: un groupe phényle ou un groupe naphthyle, ces deux groupes pouvant éventuellement être substitués par un ou plusieurs atomes ou groupes choisis indépendamment les uns des autres parmi les atomes ou groupes suivants : halogène, (C₁-C₆)alkyle, (C₃-C₇)cycloalkyle, (C₃-C₇)cycloalkyl(C₁-C₃)alkylène, halo(C₁-C₆)alkyle, (C₁-C₆)alcoxy, (C₃-C₇)cycloalcoxy, (C₃-C₇)cycloalkyl(C₁-C₃)alkylène-oxy, halo(C₁-C₆)alcoxy, (C₁-C₆)thioalkyle, -S(O)(C₁-C₆)alkyle,-S(O)₂(C₁-C₆-alkyle), hydroxyle, cyano, nitro, hydroxy(C₁-C₆)alkylène, NRaRb(C₁-C₆)alkylène, (C₁-C₆)alcoxy(C₁-C₆)alkylène-oxy, NRaRb, CONRaRb, SO₂NRaRb, NRcCORd, OC(O)NRaRb, NRcC(O)ORe, NRcSO₂Re, aryl(C₁-C₆)alkylène, aryle ou hétéroaryle, l'aryle ou l'hétéroaryle étant éventuellement substitué par un ou plusieurs substituants choisis parmi un halogène, un groupe (C₁-C₆)alkyle, (C₃-C₇)cycloalkyle, (C₃-C₇)cycloalkyl-(C₁-C₃)alkylène, halo(C₁-C₆)alkyle, (C₁-C₆)alcoxy, halo(C₁-C₆)alcoxy, NRaRb, hydroxyle, nitro ou cyano ;
- R₂ et R₃: représentent, indépendamment l'un de l'autre,
un atome d'hydrogène,
un groupe (C₁-C₆)alkyle, (C₃-C₇)cycloalkyle ou (C₃-C₇)cycloalkyl(C₁-C₃)alkylène, ce groupe étant éventuellement substitué par un groupe Rf ;
un groupe aryle, l'aryle étant éventuellement substitué par un ou plusieurs substituants choisis parmi un halogène, un groupe (C₁-C₆)alkyle, (C₃-C₇)cycloalkyle, (C₃-C₇)cycloalkyl(C₁-C₃)alkylène, halo(C₁-C₆)alkyle, (C₁-C₆)alcoxy, halo(C₁-C₆)alcoxy, NRaRb, hydroxyle, nitro ou cyano ;
- R₄ représente :: un atome d'hydrogène,
un groupe (C₁-C₆)alkyle, (C₃-C₇)cycloalkyle ou (C₃-C₇)cycloalkyl(C₁-C₃)alkylène, ce groupe étant éventuellement substitué par un groupe Rf ;
un groupe aryle, l'aryle étant éventuellement substitué par un ou plusieurs substituants choisis parmi un halogène, un groupe (C₁-C₆)alkyle, (C₃-C₇)cycloalkyle, (C₃-C₇)cycloalkyl(C₁-C₃)alkylène, halo(C₁-C₆)alkyle, (C₁-C₆)alcoxy, (C₃-C₇)cycloalcoxy, (C₃-C₇)cycloalkyl(C₁-C₃)alkylène-oxy, halo(C₁-C₆)alcoxy, NRaRb, hydroxyle, nitro, cyano, (C₁-C₆)alkyl(CO)-, CONRaRb, NRcCORd, OC(O)NRaRb, NRcC(O)ORe ou aryle, l'aryle étant éventuellement substitué par un ou plusieurs substituants choisis parmi un halogène, un groupe (C₁-C₆)alkyle, (C₃-C₇)cycloalkyle, (C₃-C₇)cycloalkyl(C₁-C₃)alkylène, halo(C₁-C₆)alkyle, (C₁-C₆)alcoxy, halo(C₁-C₆)alcoxy, NRaRb, hydroxyle, nitro ou cyano ;
- Ra et Rb: représentent, indépendamment l'un de l'autre,
un atome d'hydrogène ou un groupe (C₁-C₆)alkyle, (C₃-C₇)cycloalkyle, (C₃-C₇)cycloalkyl(C₁-C₃)alkylène, aryl(C₁-C₆)alkylène ou aryle ;
- ou Ra et Rb: forment ensemble, avec l'atome d'azote qui les porte, un groupe azétidine, pyrrolidine, pipéridine, azépine, morpholine, thiomorpholine, pipérazine, homopipérazine, ce groupe étant éventuellement substitué par un groupe (C₁-C₆)alkyle, (C₃-C₇)cycloalkyle, (C₃-C₇)cycloalkyl(C₁-C₆)akylène, aryle ou aryl(C₁-C₆)alkylène ;
- Rc et Rd: représentent, indépendamment l'un de l'autre,
un atome d'hydrogène ou un groupe (C₁-C₆)alkyle, (C₃-C₇)cycloalkyle, (C₃-C₇)cycloalkyl(C₁-C₃)alkylène, aryl(C₁-C₆)alkylène, aryle ;
ou Rc et Rd forment ensemble un groupe (C₂-C₅)alkyléne ;
- Re: représente
un groupe (C₁-C₆)alkyle, (C₃-C₇)cycloalkyle, (C₃-C₇)cycloalkyl(C₁-C₃)alkylène, aryl(C₁-C₆)alkylène, aryle ;
ou Rc et Re forment ensemble un groupe (C₂-C₅)alkylène ;
- Rf: représente
un atome d'halogène, un groupe (C₁-C₆)alcoxy, halo(C₁-C₆)alcoxy, (C₃-C₇)cycloalcoxy, (C₃-C₇)cycloalkyl(C₁-C₆)alkylène-oxy, hydroxyle, cyano, NRaRb, C(O)NRaRb, NRcCORd, OC(O)NRaRb, NRcCOORe, SO₂NRaRb, NRcSO₂Re, aryl(C₁₋C₆)alkylène, aryle, l'aryle étant éventuellement substitué par un ou plusieurs substituants choisi parmi un halogène, un groupe (C₁-C₆)alkyle, (C₃-C₇)cycloalkyle, (C₁-C₇)cycloalkyl(C₁-C₃)alkylène, halo(C₁-C₆)alkyle, (C₁-C₆)alcoxy, halo(C₁-C₆)alcoxy, NRaRb, hydroxyle, nitro ou cyano ;
à l'état de base ou de sel d'addition à un acide.

Les composés de formule (I) peuvent comporter un ou plusieurs atomes de carbone asymétriques. Ils peuvent donc exister sous forme d'énantiomères ou de diastéréoisomères. Ces énantiomères, diastéréoisomères, ainsi que leurs mélanges, y compris les mélanges racémiques, font partie de l'invention.

Dans les différents groupes tel que définis ci-dessous les groupes Ra, Rb, Rc, Rd, Re et Rf ont les mêmes définitions que celles mentionnées ci-dessus.

Parmi les composés de formule (I) objets de l'invention, un premier groupe de composés à l'état de base ou de sel d'addition à un acide est constitué des composés pour lesquels :
R₁ représente un groupe naphthyle ou un groupe phényle pouvant éventuellement être substitué par un ou plusieurs atomes ou groupes choisis indépendamment les uns des autres parmi les atomes ou groupes suivants : halogène, (C₁-C₆)alkyle, (C₃-C₇)cycloalkyle, (C₃-C₇)cycloalkyl(C₁-C₆)alkylène, (C₁-C₆)alcoxy, (C₃-C₇)cycloalkyl(C₁-C₆)alcoxy, hydroxy, cyano, nitro, NRaRb, hydroxy(C₁-C₆)alkyle, (C₁-C₆)alcoxy(C₁-C₆)alkyle, halo(C₁-C₆)alkyle, halo(C₁-C₆)alcoxy ou aryle, l'aryle étant éventuellement substitué par un ou plusieurs substituants choisis parmi un halogène, un groupe (C₁-C₆)alkyle, (C₃-C₇)cycloalkyle, (C₃-C₇)cycloalkyl-(C₁-C₃)-alkylène, halo(C₁-C₆)alkyle, (C₁-C₆)alcoxy, halo(C₁-C₆) alcoxy, NRaRb, hydroxyle, nitro ou cyano.

Parmi les composés de formule (I) objets de l'invention, un second groupe de composés à l'état de base ou de sel d'addition à un acide est constitué des composés pour lesquels :
- R₂ et R₃: représentent, indépendamment l'un de l'autre,
un atome d'hydrogène,
un groupe (C₁-C₆)alkyle, (C₃-C₇)cycloalkyle ou (C₃-C₇)cycloalkyl(C₁-C₃)alkylène éventuellement substitué par un groupe Rf ;
- R₄ représente :: un atome d'hydrogène,
un groupe (C₁-C₆)alkyle, (C₃-C₇)cycloalkyle ou (C₃-C₇)cycloalkyl(C₁-C₃)alkylène éventuellement substitué par un groupe Rf,
un groupe aryle éventuellement substitué par un ou plusieurs substituants choisis parmi un halogène, un groupe (C₁-C₆)alkyle, (C₃-C₇)cycloalkyle, (C₃-C₇)cycloalkyl(C₁-C₃)alkylène, halo(C₁-C₆)alkyle, (C₁-C₆)alcoxy, (C₃-C₇)cycloalcoxy, (C₃-C₇)cycloalkyl(C₁-C₃)alkylène-oxy, halo(C₁-C₆)alcoxy, NRaRb, hydroxyle, nitro, cyano , (C₁-C₆)alkyl(CO)-, NRcCORd, ou aryle, l'aryle étant éventuellement substitué par un ou plusieurs substituants choisis parmi un halogène, un groupe (C₁-C₆)alkyle, (C₃-C₇)cycloalkyle, (C₃-C₇)cycloalkyl(C₁-C₃)alkylène, halo(C₁-C₆)alkyle, (C₁-C₆)alcoxy, halo(C₁-C₆)alcoxy, NRaRb, hydroxyle,nitro ou cyano.

Parmi les composés de formule (I) objets de l'invention, un troisième groupe de composés à l'état de base ou de sel d'addition à un acide est constitué des composés pour lesquels :
le substituant 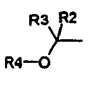 est en position méta sur le phényle.

Parmi les composés de formule (I) objets de l'invention, un quatrième groupe de composés à l'état de base ou de sel d'addition à un acide est constitué des composés pour lesquels :
R₄ représente un atome d'hydrogène et
R₂, R₃ représentent, indépendamment l'un de l'autre, un atome hydrogène, un groupe (C₁-C₆)alkyle, (C₃-C₇)cycloalkyle ou (C₃-C₇)cycloalkyl(C₁-C₃)alkylène éventuellement substitué par un groupe Rf.

Parmi les composés de formule (I) objets de l'invention, un cinquième groupe de composés à l'état de base ou de sel d'addition à un acide est constitué des composés pour lesquels :
R₁ représente un groupe naphthyle ou un groupe phényle pouvant éventuellement être substitué par un ou plusieurs atomes ou groupes choisis indépendamment les uns des autres parmi les atomes ou groupes suivants : halogène, (C₁-C₆)alkyle, (C₃-C₇)cycloalkyle, (C₃-C₇)cycloalkyl(C₁-C₆)alkylène, (C₁-C₆)alcoxy, (C₃-C₇)cycloalkyl(C₁-C₆)alcoxy, hydroxy, cyano, nitro, NRaRb, hydroxy(C₁-C₆)alkyle, (C₁-C₆)alcoxy(C₁-C₆)alkyle, halo(C₁-C₆)alkyle, halo(C₁-C₆)alcoxy ou aryle, l'aryle étant éventuellement substitué par un ou plusieurs substituants choisis parmi un halogène, un groupe (C₁-C₆)akyle, (C₃-C₇)cycloalkyle, (C₃-C₇)cycloalkyl-(C₁-C₃)-alkylène, halo(C₁-C₆)alkyle, (C₁-C₆)alcoxy, halo(C₁-C₆)alcoxy, NRaRb, hydroxyle, nitro ou cyano ;

- R₂ et R₃: représentent, indépendamment l'un de l'autre,
un atome d'hydrogène,
un groupe (C₁-C₆)alkyle, (C₃-C₇)cycloalkyle ou (C₃-C₇)cyloalkyl(C₁-C₃)alkyléne, ce groupe étant éventuellement substitué par un groupe Rf ;
un groupe aryle, l'aryle étant éventuellement substitué par un ou plusieurs substituants choisis parmi un halogène, un groupe (C₁-C₆)alkyle, (C₃-C₇)cycloalkyle, (C₃-C₇)cycloalkyl(C₁-C₃)alkylène, halo(C₁-C₆)alkyle, (C₁-C₆)alcoxy, halo(C₁-C₆)alcoxy, NRaRb, hydroxyle, nitro ou cyano ;
- R₄ représente :: un atome d'hydrogène,
un groupe (C₁-C₆)alkyle, (C₃-C₇)cycloalkyle ou (C₃-C₇)cycloalkyl(C₁-C₃)alkylène, ce groupe étant éventuellement substitué par un groupe Rf ,
un groupe aryle, l'aryle étant éventuellement substitué par un ou plusieurs substituants choisis parmi un halogène, un groupe (C₁-C₆)alkyle, (C₃-C₇)cycloalkyle, (C₃-C₇)cycloalkyl(C₁-C₃)alkylène, halo(C₁-C₆)alkyle, nitro, cyano, (C₁-C₆)alkyl(CO)-, NRcCORd.

Parmi les composés de formule (I) objets de l'invention, un sixième groupe de composés à l'état de base ou de sel d'addition à un acide est constitué des composés pour lesquels :
R₁ représente un groupe naphthyle ou un groupe phényle pouvant éventuellement être substitué par un ou plusieurs atomes ou groupes choisis indépendamment les uns des autres parmi les atomes ou groupes suivants : halogène, (C₁-C₆)alkyle, (C₃-C₇)cycloalkyle, (C₃-C₇)cycloalkyl(C₁-C₆)alkylène, (C₁-C₆)alcoxy, (C₃-C₇)cyloalkyl(C₁-C₆)alcoxy, hydroxy, cyano, nitro, NRaRb, hydroxy(C₁-C₆)alkyle, (C₁-C₆)alcoxy(C₁-C₆)alkyle, halo(C₁-C₆)alkyle, halo(C₁-C₆)alcoxy,
   - R₂ et R₃: représentent, indépendamment l'un de l'autre,
   un atome d'hydrogène,
   un groupe (C₁-C₆)alkyle, (C₃-C₇)cycloalkyle ou (C₃-C₇)cycloalkyl(C₁-C₃)alkylène, ce groupe étant éventuellement substitué par un groupe Rf ;
   un groupe aryle, l'aryle étant éventuellement substitué par un ou plusieurs substituants choisis parmi un halogène, un groupe (C₁-C₆)alkyle, (C₃-C₇)cycloalkyle, (C₃-C₇)cycloalkyl(C₁-C₃)alkyléne, halo(C₁-C₆)alkyle, (C₁-C₆)alcoxy, halo(C₁-C₆)alcoxy, NRaRb, hydroxyle, nitro ou cyano ;
   - R₄ représente :: un atome d'hydrogène,
   un groupe (C₁-C₆)alkyle, (C₃-C₇)cycloalkyle ou (C₃-C₇)cycloalkyl(C₁-C₃)alkylène, ce groupe étant éventuellement substitué par un groupe Rf ,
   un groupe aryle, l'aryle étant éventuellement substitué par un ou plusieurs substituants choisis parmi un halogène, un groupe (C₁-C₆)alkyle, (C₃-C₇)cycloalkyle, (C₃-C₇)cycloalkyl(C₁-C₃)alkylène, halo(C₁-C₆)alkyle, nitro, cyano, (C₁-C₆)alkyl(CO)-, NRcCORd.

Les combinaisons des groupes un à six tels que définis ci dessus font également partie de l'invention.

Les composés de formule (I) peuvent exister à l'état de bases ou de sels d'addition à des acides. De tels sels d'addition font partie de l'invention.

Ces sels peuvent être préparés avec des acides pharmaceutiquement acceptables, mais les sels d'autres acides utiles, par exemple, pour la purification ou l'isolement des composés de formule (I) font également partie de l'invention.

Les composés de formule (I) peuvent également exister sous forme d'hydrates ou de solvats, à savoir sous forme d'associations ou de combinaisons avec une ou plusieurs molécules d'eau ou avec un solvant. De tels hydrates et solvats font également partie de l'invention.

Dans le cadre de la présente invention, on entend par :
- un atome d'halogène : un fluor, un chlore, un brome ou un iode ;
- un groupe (C₁-C₆)alkyle : un groupe aliphatique de 1 à 6 carbones saturé, linéaire ou ramifié. A titre d'exemples, on peut citer les groupes méthyle, éthyle, propyle, isopropyle, butyle, isobutyle, tertbutyle, pentyle etc ;
- un groupe (C₃-C₇)cycloalkyle : un groupe carboné cyclique de 3 à 7 carbones. A titre d'exemples, on peut citer les groupes cyclopropyle, cyclobutyle, cyclopentyle, cyclohexyle, etc ;
- un alkylène : un groupe alkyle divalent saturé, linéaire ou ramifié, par exemple un groupe (C₁-C₆)alkylène représente une chaîne carbonée divalente de 1 à 6 atomes de carbone, linéaire ou ramifiée, plus particulièrement un méthylène, éthylène, 1-méthyléthylène, propylène ;
- un groupe (C₁-C₆)alcoxy : un radical -O-alkyle où le groupe alkyle est tel que précédemment défini ;
- un groupe (C₃-C₇)cycloalcoxy : un radical -O-cycloalkyle où le groupe cycloalkyle est tel que précédemment défini ;
- un groupe halo(C₁-C₆)alkyle : un groupe aliphatique de 1 à 6 carbones saturé, linéaire, ramifié ou cyclique étant substitué par un ou plusieurs atomes d'halogène identiques ou différents. A titre d'exemples, on peut citer les groupes CF3, CH2CF3, CHF2, CCl₃.
- un groupe halo(C₁-C₆)alcoxy : un radical -O-alkyle où le groupe alkyle est tel que précédemment défini et qui est substitué par un ou plusieurs atomes d'halogène identiques ou différents. A titre d'exemples, on peut citer les groupes OCF3, OCHF2, OCCl₃.
- Un groupe thioalkyle : un radical S-alkyle ou où le groupe alkyle est tel que précédemment défini
- les atomes de soufre et d'azote peuvent être à l'état oxydé (N-oxide, sulfoxide, sulfone)
- un aryle : un groupe aromatique cyclique comprenant entre 6 et 10 atomes de carbone. A titre d'exemples de groupes aryles, on peut citer les groupes phényle ou naphthyle ;
- un hétéroaryle : un groupe cyclique aromatique de 5 à 10 chaînons contenant de 1 à 4 hétéroatomes choisis parmi O, S ou N. A titre d'exemple non limitatif, on peut citer les groupes imidazolyle, thiazolyle, oxazolyle, furanyle, thiophényle, oxadiazolyle, tétrazolyle, pyridinyle, pyrazinyle, pyrimidinyle, pyridazinyle, indolyle, benzofuranyle, benzothiophényle, benzoxazolyle, benzimidazolyle, indazolyle, , benzothiazolyle, isobenzothiazolyle, benzotriazolyle, quinoléinyle, isoquinoléinyle, quinoxalinyle.

Parmi les composés de formule (I) objets de l'invention, on peut notamment citer les composés suivants :
[4-(2-Phénylimidazo[1,2-*a*]pyridin-6-yl)phényl]méthanol
[3-(2-Phénylimidazo[1,2-*a*]pyridin-6-yl)phényl]méthanol
{4-[2-(4-Pyrrolidin-1-ylphényl)-imidazo[1,2-*a*]pyridin-6-yl]phényl}méthanol
[4-(2-Biphényl-4-ylimidazo[1,2-*a*]pyridin-6-yl)phényl]méthanol
3-[6-(4-Hydroxyméthylphényl)imidazo[1,2-*a*]pyridin-2-yl]benzonitrile
3-[6-(3-Hydroxyméthylphényl)imidazo[1,2-*a*]pyridin-2-yl]benzonitrile
3-{6-[3-(2-Méthoxyéthoxyméthyl)phényl]imidazo[1,2-*a*]pyridin-2-yl}benzonitrile
4-[6-(3-Hydroxyméthylphényl)imidazo[1,2-*a*]pyridin-2-yl]benzonitrile
[3-[2-(Naphthalèn-2-yl)imidazo[1,2-*a*]pyridin-6-yl]phényl]méthanol
[3-(2-*p*-Tolylimidazo[1,2-*a*]pyridin-6-yl)phényl]méthanol
{3-[2-(4-Pyrrolidin-1-ylphényl)imidazo[1,2-*a*]pyridin-6-yl]phényl}méthanol
{3-[2-(3-Trifluorométhoxyphényl)imidazo[1,2-*a*]pyridin-6-yl]phényl}méthanol
{3-[2-(4-Nitrophényl)imidazo[1,2-*a*]pyridin-6-yl]phényl}méthanol
{4-[2-(4-Diéthylaminophényl)imidazo[1,2-*a*]pyridin-6-yl]phényl}méthanol
3-(2-Naphthalèn-1-ylimidazo[1,2-*a*]pyridin-6-yl)phényl]méthanol
{3-[2-(2,4-Diméthylphényl)imidazo[1,2-*a*]pyridin-6-yl]phényl}méthanol
6-[3-(2-Méthoxyéthoxyméthyl)phényl]-2-naphthalèn-2-ylimidazo[1,2-*a*]pyridine
{3-[2-(4-Méthoxyphényl)imidazo[1,2-*a*]pyridin-6-yl]phényl}méthanol
{3-[2-(3-Méthoxyphényl)imidazo[1,2-*a*]pyridin-6-yl]phényl}méthanol
{3-[2-(2-Méthoxyphényl)imidazo[1,2-*a*]pyridin-6-yl]phényl}méthanol
{3-[2-(2,4-Diméthoxyphényl)imidazo[1,2-*a*]pyridin-6-yl]phényl}méthanol
{3-[2-(4-Fluorophényl)imidazol[1,2-*a*]pyridin-6-yl]phényl} méthanol
{3-[2-(3,5-Difluorophényl)imidazo[1,2-*a*]pyridin-6-yl]phényl}méthanol
{3-[2-(3-Fluoro-5-trifluorométhylphényl)imidazo[1,2-*a*]pyridin-6-yl]phényl} méthanol
{3-[2-(4-Chlorophényl)imidazo[1,2-*a*]pyridin-6-yl]phényl}méthanol
Chlorhydrate (1:1) de {3-[2-(4-Chlorophényl)imidazo[1,2-*a*]pyridin-6-yl]phényl}méthanol
{3-[2-(3-Chlorophényl)imidazo[1,2-*a*]pyridin-6-yl]phényl}méthanol
{3-[2-(4-Chloro-3-méthylphényl)imidazo[1,2-*a*]pyridin-6-yl]phényl}méthanol
{3-[2-(3,4-Dichlorophényl)imidazo[1,2-*a*]pyridin-6-yl]phényl}méthanol
{3-[2-(3-Fluorophényl)imidazo[1,2-*a*]pyridin-6-yl]phényl}méthanol
6-[3-(4-Chlorophénoxyméthyl)phényl]-2-*p*-tolylimidazo[1,2-*a*]pyridine
*N-*{4-[3-(2-*p*-Tolylimidazo[1,2-*a*]pyridin-6-yl)benzyloxy]phényl}acétamide
6-[3-(3-Nitrophénoxyméthyl)phényl]-2-*p*-tolylimidazo[1,2-*a*]pyridine
6-[3-(4-Méthylphénoxyméthyl)phényl]-2-*p*-tolylimidazo[1,2-*a*]pyridine
4-[3-(2-*p*-Tolylimidazo[1,2-*a*]pyridin-6-yl)benzyloxy]benzonitrile
1-{4-[3-(2-*p*-Tolylimidazo[1,2-*a*]pyridin-6-yl)benzyloxy]phényl}éthanone
6-[3-(4-Trifluorométhylphénoxyméthyl)phényl]-2-*p*-tolylimidazo[1,2-*a*]pyridine
{3-[2-(3-Fluoro-4-méthylphényl)imidazo[1,2-*a*]pyridin-6-yl]phényl}méthanol
2-{3-[2-(4-Chlorophényl)imidazo[1,2-*a*]pyridin-6-yl]phényl}propan-2-ol
{2-[2-(4-Chlorophényl)imidazo[1,2-*a*]pyridin-6-yl]phényl}méthanol
Chlohydrate (1:1) de {2-[2-(4-Chlorophényl)imidazo[1,2-*a*]pyridin-6-yl]phényl}méthanol
1-{3-[2-(4-Chlorophényl)imidazo[1,2-*a*]pyridin-6-yl]phényl}éthanol racémique
{3-[2-(2,4-Dichlorophényl)imidazo[1,2-*a*]pyridin-6-yl]phényl}méthanol
{3-[2-(2,4-Difluorophényl)imidazo[1,2-*a*]pyridin-6-yl]phényl}méthanol
{3-[2-(3,4-Difluorophényl)imidazo[1,2-*a*]pyridin-6-yl]phényl}méthanol
Chlohydrate (1 :1) de {3-[2-(3,4-Difluorophényl)imidazo[1,2-*a*]pyridin-6-yl]phényl}méthanol
{3-[2-(2-Chlorophényl)imidazo[1,2-*a*]pyridin-6-yl]phényl} méthanol
{3-[2-(4-Trifluorométhylphényl)imidazo[1,2-*a*]pyridin-6-yl]phényl}méthanol
{3-[2-(4-(Difluorométhyl)phényl)imidazo[1,2-*a*]pyridin-6-yl]phényl}méthanol
1-{2-[2-(4-Chlorophényl)imidazo[1,2-*a*]pyridin-6-yl]phényl}éthanol racémique
2-(4-Chlorophényl)-6-(2-méthoxyméthylphényl)imidazo[1,2-*a*]pyridine
Chlohydrate (1 : 1) de 2-(4-Chlorophényl)-6-(2-méthoxyméthylphényl)imidazo[1,2-*a*]pyridine
2-(4-Chlorophényl)-6-(4-méthoxyméthylphényl)imidazo[1,2-*a*]pyridine
1-{3-[2-(4-Chlorophényl)imidazo[1,2-*a*]pyridin-6-yl]phényl}propan-1-ol racémique
1-{3-[2-(4-Chlorophényl)imidazo[1,2-*a*]pyridin-6-yl]phényl}pentan-1-ol racémique
1-{3-[2-(4-Chlorophényl)imidazo[1,2-*a*]pyridin-6-yl]phényl}heptan-1-ol racémique
1-{3-[2-(4-Chlorophényl)imidazo[1,2-*a*]pyridin-6-yl]phényl}-3-méthylbutan-1-ol racémique
{3-[2-(4-Chlorophényl)imidazo[1,2-*a*]pyridin-6-yl]phényl}cyclopentylméthanol racémique
{3-[2-(4-Chlorophényl)imidazo[1,2-*a*]pyridin-6-yl]phényl}phenylméthanol racémique
Enantiomère dextrogyre de 1-{3-[2-(4-Chlorophényl)imidazo[1,2-*a*]pyridin-6-yl]phényl}éthanol
Enantiomère lévogyre de 1-{3-[2-(4-Chlorophényl)imidazo[1,2-*a*]pyridin-6-yl]phényl}éthanol
2-(4-Chtorophényl)-6-(3-méthoxyméthylphényl)imidazo[1,2-*a*]pyridine
1- {4-[2-(4-Chlorophényl)imidazo[1,2-*a*]pyridin-6-yl]phényl}éthanol racémique
1-{3-[2-(2,4-Difluorophényl)imidazo[1,2-*a*]pyridin-6-yl]phényl}éthanol racémique
1-{3-[2-(4-Fluororophényl)imidazo[1,2-*a*]pyridin-6-yl]phényl}éthanol racémique
1-{3-[2-(3,4-Difluorophényl)imidazo[1,2-*a*]pyridin-6-yl]phényl}éthanol racémique
1-{3-[2-(2-Chlorophényl)imidazo[1,2-*a*]pyridin-6-yl]phényl}éthanol racémique
1-{3-[2-(3-Fluorophényl)imidazo[1,2-*a*]pyridin-6-yl]phényl}éthanol racémique
1-{3-[2-(3-Chlorophényl)imidazo[1,2-*a*]pyridin-6-yl]phényl}éthanol racémique
4-[6-(3-Hydroxyméthylphényl)imidazo[1,2-*a*]pyridin-2-yl]phénol

Conformément à l'invention, on peut préparer les composés de formule générale (I) selon le procédé décrit dans le schéma 1.

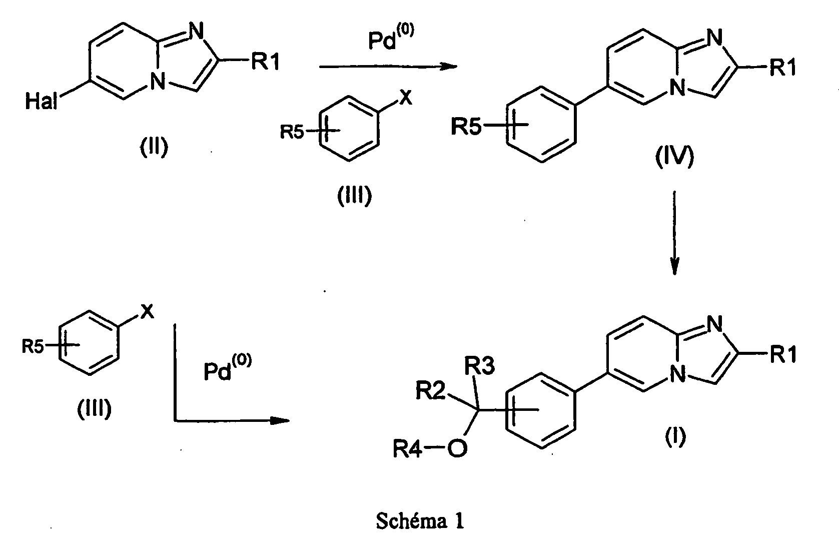

On peut préparer les composés de l'invention suivant le schéma 1 par une réaction de couplage, catalysée par un métal tel que le palladium, entre une 2-arylimidazopyridine de formule générale (II), dans laquelle R1 est défini comme précédemment et Hal représente un atome d'halogène, et un dérivé de formule générale (III) dans laquelle X représente un dérivé de bore ou d'étain et R5 représente le groupe 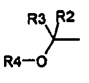 pour obtenir les composés de formule générale (I).

On peut aussi préparer les composés de l'invention suivant le schéma 1 par une réaction de couplage, catalysée par un métal tel que le palladium, entre une 2-arylimidazopyridine de formule générale (II), dans laquelle R1 est défini comme précédemment et Hal représente un atome d'halogène, et un dérivé de formule générale (III) dans laquelle X représente un dérivé de bore ou d'étain et R5 représente un dérivé carbonylé R₂COR₃ , dans lequel R2 et R3 sont définis comme précédemment pour obtenir les composés de formule générale (IV) par exemple selon la méthode décrite par A. Gueiffier dans Helv. Chim. Acta 2001, 84, 3610-3615.

Ensuite, on peut transformer les composés de formule générale (IV) en composés de formule générale (I) par action d'un dérivé organométallique tel qu'un organomagnésien, ou par réduction du groupement carbonyle au moyen d'un hydrure métallique, par exemple le borohydrure de sodium ou un de ses dérivés, ou tout autre méthode connue de l'homme de l'art.

Les produits de formule (I), peuvent être soumis, si désiré et si nécessaire, à toutes réactions connues de l'homme de l'art, dans un ordre quelconque, pour être transformés en d'autres produits de formule (I),
A titre d'exemples de réactions, on peut citer : les réactions d'estérification ou d'amidification de fonction acide, les réactions de carbamoylation, les réactions d'hydrolyse de fonction ester, les réactions de transformation de fonction hydroxyle en fonction alcoxy, les réactions de couplage catalysées par un métal de transition, les réactions de protection des fonctions réactives, les réaction d'élimination des groupements protecteurs que peuvent porter les fonctions réactives protégées, les réactions de salification par un acide minéral ou organique ou par une base pour obtenir le sel correspondant, les réactions de dédoublement des formes racémiques en énantiomères, lesdits produits de formule (I) ainsi obtenus étant le cas échéant sous toutes les formes isomères possibles racémiques, énantiomères et diastéréoisomères.

Dans le schéma 1, les composés de départ et les réactifs, quand leur mode de préparation n'est pas décrit, sont disponibles dans le commerce ou décrits dans la littérature, ou bien peuvent être préparés selon des méthodes qui y sont décrites ou qui sont connues de l'Homme du métier.

Les exemples suivants décrivent la préparation de certains composés conformes à l'invention. Ces exemples ne sont pas limitatifs et ne font qu'illustrer la présente invention. Les numéros des composés exemplifiés renvoient à ceux donnés dans les tableaux ci-après, qui illustrent les structures chimiques et les caractéristiques physiques de quelques composés selon l'invention.

La nomenclature des composés a été établie à partir du logiciel Autonom.

### Exemple 1 : [4-(2-Phénylimidazo[1,2-a]pyridin-6-yl)phényl]méthanol (composé 1 du tableau)

### 1.1 6-Bromo-2-phénylimidazo[1,2-a]pyridine

On place dans un ballon 1,99 g de 2-bromo-1-phényléthanone, 1,73 g de 2-amino-5-bromopyridine et 1 g d'hydrogénocarbonate de sodium dans un mélange de 20 ml d'éthanol et 5 ml d'eau. On chauffe à 80°C pendant 4h, laisse refroidir et ajoute 40 ml d'eau. Le mélange est agité pendant 15 mn, puis le précipité est recueilli par filtration ; on le lave à l'eau, puis à l'éther diisopropylique, le sèche au dessicateur. On obtient 1,8 g de composé. F = 192 -194°C.

### 1.2 [4-(2-Phénylimidazo[1,2-a]pyridin-6-yl)phényl]méthanol

150 mg de 6-bromo-2-phénylimidazo[1,2-*a*]pyridine, 125 mg d'acide 4-(hydroxyméthyl)phénylboronique, 19 mg de tétrakis(triphénylphosphine)palladium et 2 ml d'acétonitrile sont placés dans un tube à micro-ondes. Sous un courant d'azote, on y ajoute 2 ml de toluène dégazé à l'azote puis 2 ml d'une solution 2M de carbonate de sodium. Le tube est placé dans un appareil à micro-ondes et irradié à 150°C pendant 15 mn. La phase organique est recueillie, séchée, puis concentré sous pression réduite. Le résidu est repris par de l'éther diisopropylique, le précipité est recueilli par filtration, lavé, séché. On le purifie par recristallisation dans le n-butanol. On obtient 52 mg de composé. F = 238 - 240°C. RMN 1H (DMSO-d6,δ en ppm) : 4,54 (d, J = 5,5Hz, 2H) ; 5,2 (t, J = 5,6Hz, 1H) ; de 7,24 à 7,73 (m, 9H) ; 7,96 (m, 2H) ; 8,36 (s, 1H); 8,84 (t, J = 1,3Hz, 1H). M+H = 301.

### Exemple 2 : [3-(2-(Naphthalèn-2-yl)imidazo[1,2-a]pyridin-6-yl)phényl]méthanol (composé 9 du tableau)

### 2.1 6-Bromo-2-(naphthalèn-2-yl)imidazo[1,2-a]pyridine

En procédant comme dans l'exemple 1, et en partant de 0,5 g de 2-bromo-1-(naphthalèn-2-yl)éthanone, de 0,72 g de 2-amino-5-bromopyridine et de 0,29 g d'hydrogénocarbonate de sodium, on obtient 0,83 g de 6-bromo-2-(naphthalèn-2-yl)imidazo[1,2-*a*]pyridine. F = 226 - 228°C.

### 2.2 [3-(2-(Naphthalèn-2-yl)imidazo[1,2-a]pyridin-6-yl)phényl]méthanol

Sous un courant d'azote, 500 mg de 6-bromo-2-(napthalèn-2-yl)imidazo[1,2-*a*]pyridine, 235 mg d'acide 3-(hydroxyméthyl)phénylboronique et 90 mg de tétrakis(triphénylphosphine)palladium sont placés dans un tube à micro-ondes contenant 5 ml de toluène préalablement dégazé sous courant d'azote, 5 ml d'acétonitrile et 6 ml d'une solution 0,5M de carbonate de sodium. Le tube est placé dans un appareil à micro-ondes et irradié à 150°C pendant 15 mn. La phase organique est séparée,séchée et le filtrat est concentré sous pression réduite. Le résidu obtenu est repris par 5 ml de dichlorométhane. Il se forme un précipité que l'on recueille par filtration, lave au dichlorométhane puis à l'éther diisopropylique et sèche sous pression réduite. On répète l'expérience plusieurs fois (6) et les solides sont réunis. On obtient 2,2 g de composé que l'on recristallise dans un mélange n-propanol/eau 1/1. Le précipité est essoré, lavé à l'éther diisopropylique et séché sous pression réduite. On obtient 1,96 g de composé. F = 161 - 163°C. RMN 1H (DMSO-d6, δ en ppm) : 4,58 (d, J = 5,5Hz, 2H); 5,24 (t, J = 5,7Hz, 1H) ; de 7,29 à 7,73 (m, 8H) ; de 7,86 à 8,13 (m, 4H) ; 8,52 (m, 2H) ; 8,84 (m, 1H). M+H = 351.

### Exemple 3: [3-(2-p-Tolylimidazo[1,2-a]pyridin-6-yl)phényl]méthanol (composé 10 du tableau)

### 3.1 6-Bromo-2-p-tolylimidazo[1,2-a]pyridine

En procédant comme dans l'exemple 1, en partant de 150 mg de 2-bromo-1-*p*-tolyléthanone, de 185 mg de 2-amino-5-bromopyridine et de 87 mg d'hydrogénocarbonate de sodium, on obtient 200 mg de 6-bromo-2-*p*-tolylimidazo[1,2-*a*]pyridine. Le composé est purifié par chromatographie sur gel de silice, en éluant avec un mélange dichlorméthane/méthanol 98/2. On obtient 60 mg de composé. F = 226 - 228°C.

### 3.2 [3-(2-p-Tolylimidazo[1,2-a]pyridin-6-yl)phényl]méthanol

Sous un courant d'azote, 200 mg de 6-bromo-2-*p*-tolylimidazo[1,2-*a*]pyridine, 160 mg d'acide 3-(hydroxyméthyl)phénylboronique et 24 mg de tétrakis(triphénylphosphine)palladium sont placés dans un tube à micro-ondes contenant 2 ml de toluène préalablement dégazé sous courant d'azote, 2 ml d'acétonitrile et 2 ml d'une solution 2M de carbonate de sodium. Le tube est placé dans un appareil à micro-ondes et irradié à 150°C pendant 15 mn. La phase organique est séparée, séchéeet le filtrat est concentré sous pression réduite. Le résidu obtenu est repris par 6 ml de dichlorométhane. On recueille le précipité par filtration, le lave au dichlorométhane et le sèche au dessicateur sous pression réduite. On obtient 100 mg de composé. F = 171 - 173°C. RMN 1H (DMSO-d6, δ en ppm) : 2,32 (s, 3H) ; 4,57 (d, J = 5,6Hz, 2H) ; 5,23 (t, J = 5,7Hz, 1H) ; de 7,19 à 7,67 (m, 8H) ; 7,84 (d, J = 8Hz, 2H) ; 8,32 (s, 1H) ; 8,82 (m, 1H). M+H = 315.

### Exemple 4:6-[3-(2-Méthoxyéthoxyméthyl)phényl]-2-(naphthalèn-2-yl)imidazo[1,2-a]pyridine (composé 17 du tableau)

Dans un tube à pression, on dissout 100 mg de [3-(2-naphthalèn-2-ylimidazo[1,2-*a*]pyridin-6-yl)phényl]méthanol (exemple 2) et 120 mg d'éther méthylique de 2-bromoéthanol dans 5 ml d'un mélange méthanol/diméthylformamide 1/1 et y ajoute 1 g de fluorure de potassium sur alumine. Le tube est fermé et chauffé 16h à 80°C. Après refroidissement, on élimine par filtration le minéral que l'on lave au dichlorométhane. Le filtrat est concentré sous pression réduite et purifié par chromatographie sur gel de silice en éluant avec un mélange dichlorométhane/méthanol 99/1. On obtient une huile qui cristallise dans 5 ml d'éther diisopropylique. Le précipité est recueilli par filtration, lavé à l'éther diisopropylique et séché sous pression réduite. On obtient 44 mg de composé. F = 80 - 82°C. RMN 1H (DMSO-d6, δ en ppm) : 3,25 (m, 3H) 3,55 (m, 4H) ; 4,57 (s, 2H); de 7,27 à 7,80 (m, 8H) ; de 7,82 à 8,16 (m, 4H) ; 8,52 (m, 2H) ; 8,89 (m, 1H). M+H = 409.

### Exemple 5 : {3-[2-(4-Chlorophényl)imidazo[1,2-a]pyridin-6-yl]phényl}méthanol (composé 25 du tableau)

### 5.1 6-Bromo-2-(4-chlorophényl)imidazo[1,2-a]pyridine

En procédant comme dans l'exemple 1, en partant de 675 mg de 2-bromo-1-(4-chlorophényl)éthanone, de 500 mg de 2-amino-5-bromopyridine et de 290 mg d'hydrogénocarbonate de sodium, on obtient 680 mg de 6-bromo-2-(4-chlorophényl)imidazo[1,2-a]pyridine. Le composé est purifié par chromatographie sur gel de silice, en éluant avec un mélange dichlorométhane/méthanol 98/2. On obtient 60 mg de composé. F=210-211°C.

### 5.2 {3-[2-(4-Chlorophényl)imidazo[1,2-a]pyridin-6-yl]phényl}méthanol

Sous un courant d'azote, 210 mg de 6-bromo-2-(4-chlorophényl)imidazo[1,2-*a*]pyridine, 155 mg d'acide 3-(hydroxyméthyl)phénylboronique et 24 mg de tétrakis(triphénylphosphine)palladium sont placés dans un tube à micro-ondes contenant 3 ml de toluène préalablement dégazé sous courant d'azote, 3 ml d'acétonitrile et 3 ml d'une solution 2M de carbonate de sodium. Le tube est placé dans un appareil à micro-ondes et irradié à 150°C pendant 15 mn. La phase organique est séparée, séchée et le filtrat est concentré sous pression réduite. Le résidu obtenu est repris par du dichlorométhane. On recueille le précipité par filtration, le lave au dichlorométhane et le sèche au dessicateur sous pression réduite. On obtient 175 mg de composé. F = 181 - 182°C. RMN 1H (DMSO-d6, δ en ppm) : 4,57 (d, J = 5,5Hz, 2H); 5,23 (t, J = 5,6Hz, 1H) ; de 7,28 à 7,71 (m, 8H) ; 7,97 (m, J = 8,5Hz, 2H) ; 8,41 (s, 1H) ; 8,84 (m, 1H). M+H = 335.

### Exemple 6: Chlorhydrate (1: 1) de {3-[2-(4-chlorophényl)imidazo[1,2-a]pyridin-6-yl]phényl}méthanol (composé 26 du tableau)

2,37 g de {3-[2-(4-Chlorophényl)imidazo[1,2-*a*]pyridin-6-yl]phényl}méthanol sont mis en suspension dans 80 ml de dichlorométhane ; on y ajoute, goutte à goutte et sous agitation, 4,26 ml d'une solution 5N d'acide chlorhydrique dans le 2-propanol et agite à température ambiante pendant 2 heures. Le mélange réactionnel est ensuite concentré sous pression réduite. Le solide résiduel est repris par de l'éther diisopropylique, le précipité est recueilli par filtration, lavé par du dichlorométhane puis de l'acétate d'éthyle et essoré. Le solide est dissous à température ambiante par le minimum de méthanol puis reprécipité à l'aide d'éther diisopropylique. Le précipité est recueilli par filtration et séché à l'étuve sous pression réduite à 60°C. On obtient 2,23 g de solide jaune pâle. F = 244 - 246°C. RMN 1H (DMSO-d6, δ en ppm) : 4,59 (s, 2H) ; de 7,36 à 7,55 (m, 2H) ; de 7,58 à 7,74 (m, 4H); 7,96 (d, J = 9,4Hz, 1H); 8,05 (m, J = 8,7Hz, 2H); 8,14 (dd, J = 9,3Hz et 1,7Hz,1H) ; 8,70 (s, 1H) ; 9,15 (m, 1H). M+H = 335.

### Exemple 7 : 6-[3-(4-Chlorophénoxyméthyl)phényl]-2-p-tolylimidazo[1,2-a]pyridine (composé 31 du tableau)

On dissout 200 mg de [3-(2-*p*-tolylimidazo[1,2-*a*]pyridin-6-yl)phényl]méthanol (exemple 3) dans 20 ml de tétrahydrofurane sec, y ajoute 123 mg de *p*-chlorophénol, 193 mg de tributylphosphine et 210 mg de 1,1'-azodicarbonyldipipéridine et laisse le mélange agiter 16 h à température ambiante. On évapore le solvant sous pression réduite. Le résidu est repris par 10 ml d'acétate d'éthyle, on élimine le précipité, concentre le filtrat et purifie le résidu par chromatographie. Le solide obtenu est repris par de l'éther de pétrole, le précipité est recueilli par filtration et séché sous pression réduite. On obtient 52 mg de composé. F = 182 -184°C. RMN 1H (DMSO-d6, δ en ppm) : 2,32 (s, 3H) ; 5,17 (s, 2H) ; 7,06 (d, J = 7Hz, 2H) ; 7,23 (d, J = 7,2Hz, 2H) ; 7,32 (d, J = 7,3Hz, 2H) ; de 7,40 à 7,80 (m, 6H) ; 7,84 (m, J = 7,8Hz, 2H) ; 8,32 (s, 1H) ; 8,86 (m, 1H). M+H = 425.

### Exemple 8 : 2-{3-[2-(4-Chlorophényl)imidazo[1,2-a]pyridin-6-yl]phényl}propan-2-ol (composé 39 du tableau)

### 8.1 1-{3-[2-(4-Chlorophényl)imidazo[1,2-a]pyridin-6-yl]phényl}éthanone

300 mg de 6-bromo-2-(4-chlorophényl)imidazo[1,2-*a*]pyridine (préparé comme décrit en 5.1),240 mg d'acide 3-acétylphenylboronique, 34 mg de tétrakis(triphénylphosphine)palladium sont mélangés dans un tube à micro-ondes contenant 4,5 ml d'acétonitrile, 4,5 ml de toluène et 4,5 ml d'une solution 2M d'hydrogénocarbonate de sodium. Le tube est placé dans un appareil à micro-ondes et irradié à 150°C pendant 15 mn. La phase organique est séparée, séchée et concentré sous pression réduite. On obtient un résidu solide qui est trituré dans un mélange de 3 ml de dichlorométhane et de 3 ml d'éther diisopropylique pendant 30 mn. Le précipité est recueilli par filtration, lavé avec de l'éther diisopropylique et séché au dessicateur sous pression réduite. On obtient 209 mg de composé. F = 173 - 175°C.

### 8.2 2-{3-[2-(4-Chlorophényl)imidazo[1,2-a]pyridin-6-yl]phényl}propan-2-ol

Sous courant d'azote, 150 mg du composé obtenu à l'étape 8.1 est placé dans un ballon et dissous dans un mélange de 20 ml d'éther diéthylique et 10 ml de tetrahydrofurane secs. On refroidit au bain de glace et ajoute goutte à goutte 1,3 ml d'une solution 1M de bromure de méthylmagnésium dans l'éther dibutylique. On laisse le mélange agiter dans le bain de glace pendant une heure, puis ajoute 5 ml d'une solution aqueuse saturée de chlorure d'ammonium. La phase organique est séparée, séchée sur sulfate de sodium et concentré sous pression réduite. Le résidu est purifié par chromatographie sur gel de silice en éluant par un mélange dichlorométhane/méthanol 99/1. Le solide obtenu est trituré dans de l'éther diisopropylique et recueilli par filtration, puis séché au dessicateur sous pression réduite. On obtient 65 mg de composé. F = 166 - 168°C. RMN 1H (DMSO-d6, δ en ppm) : 1,47 (s, 6H) ; 5,06 (s, 1H); de 7,33 à 7,69 (m, 7H) ; 7,78 (m, 1H) ; 7,97 (m, J = 8,5Hz, 2H) ; 8,42 (s, 1H) ; 8,83 (m, 1H). M+H = 363.

### Exemple 9 : {2-[2-(4-Chlorophényl)imidazo[1,2-a]pyridin-6-yl]phényl}méthanol (composé 40 du tableau)

Sous courant d'azote on place 200 mg de 6-bromo-2-(4-chlorophényl)imidazo[1,2-*a*]pyridine (préparé comme décrit en 5.1), 148 mg d'acide 2-(hydroxyméthyl)phénylboronique, 22,5 mg de tétrakis(triphénylphosphine)palladium dans un tube à micro-ondes contenant un mélange de 3 ml d'acétonitrile, 3 ml de toluène et 3 ml d'une solution 2M d'hydrogénocarbonate de sodium. Le tube est placé dans un appareil à micro-ondes et irradié à 150°C pendant 15 mn. La phase organique est séparée, séchée et concentrée sous pression réduite. Le résidu est purifié par chromatographie sur gel de silice en éluant par un mélange dichlorométhane/méthanol 98/2. Le solide obtenu est trituré dans de l'éther diisopropylique et recueilli par filtration, puis séché au dessicateur sous pression réduite. On obtient 127 mg de composé. F = 164 - 166°C. RMN 1H (DMSO-d6, δ en ppm) : 4,44 (d, J = 5,6Hz, 2H) ; 5,19 (t, J = 5,4Hz, 1H) ; de 7,25 à 7,64 (m, 8H) ; 7,98 (m, J = 8,3Hz, 2H) ; 8,41 (s, 1H) ; 8,54 (m, 1H). M+H = 335.

### Exemple 10: Chlorhydrate (1: 1) de {2-[2-(4-chhlorophényl)imidazo[1,2-a]pyridin-6-yl]phényl}méthanol (composé 41 du tableau)

40 mg de {2-[2-(4-Chlorophényl)imidazo[1,2-*a*]pyridin-6-yl]phényl}mèthanol sont mis en suspension dans 1,5 ml d'éthanol ; on y ajoute, goutte à goutte et sous agitation, 1,79 ml d'une solution 0,1N d'acide chlorhydrique dans le 2-propanol et agite à température ambiante pendant 30 minutes. Le mélange réactionnel est ensuite concentré sous pression réduite. Le solide résiduel est repris par de l'éthanol, le précipité est recueilli par filtration, lavé par de l'éthanol puis de l'éther diéthylique et essoré. Le produit est séché à l'étuve sous pression réduite à 60°C. On obtient 17 mg de solide blanc. F = 238 - 239°C. RMN 1H (DMSO-d6, δ en ppm) : 4,50 (s, 2H) ; de 7,36 à 7,54 (m, 3H) ; de 7,60 à 7,70 (m, 3H) ; 7,77 (m, 1H) ; 7,86 (d, J = 7,7Hz, 1H) ; 8,14 (d, J = 9,2Hz, 2H) ; 8,73 (s, 1H) ; 8,86 (s, 1H). M+H = 335.

### Exemple 11 : 1-{3-[2-(4-chlorophényl)imidazo[1,2-a]pyridin-6-yl]phényl}éthanol racémique (composé 42 du tableau)

A 150 mg de 1-{3-[2-(4-Chlorophényl)imidazo[1,2-*a*]pyridin-6-yl]phényl}éthanone (composé obtenu en 8.1) dissous dans 20 ml de méthanol, on ajoute par portions 164 mg de borohydrure de sodium. Le mélange est ensuite agité à température ambiante pendant une heure, puis le solvant est évaporé sous pression réduite. Le résidu est repris entre l'eau et le dichlorométhane, la phase organique est décantée, séchée sur sulfate de sodium puis le solvant est évaporé sous pression réduite. Le résidu est trituré dans de l'éther diisopropylique et recueilli par filtration, puis séché au dessicateur sous pression réduite. On obtient 124 mg de composé. F = 174 - 176°C. RMN 1H (DMSO-d6, δ en ppm) : 1,37 (d, J = 6,5Hz, 3H) ; 4,79 (m, 1H) ; 5,19 (d, J = 4,2Hz, 1H) ; de 7,31 à 7,69 (m, 8H) ; 7,97 (m, J = 8,6Hz, 2H) ; 8,41 (s, 1H) ; 8,84 (m, 1H). M+H = 349.

### Exemple 12 : Enantiomère dextrogyre du 1-{3-[2-(4-Chlorophényl)imidazo[1,2-a]pyridin-6-yl]phényl}éthanol (composé 60 du tableau)

263 mg de 1-{3-[2-(4-Chlorophényl)imidazo[1,2-*a*]pyridin-6-yl]phényl}éthanol racémique (composé n°42, exemple 11) sont déposés sur une colonne ChiralPAK AD DAICEL 20µm 50x250mm. On élue par un mélange de n-heptane et de 2-propanol 80/20. Après cristallisation dans l'éther diisopropylique, on obtient 115 mg de composé le moins retenu. F = 168 - 170°C. [α]_{D} = +14,3° (c = 0,4 ; MeOH). RMN 1H (DMSO-d6, δ en ppm) : 1,36 (d, J = 6,2Hz, 3H) ; 4,79 (m, 1H) ; 5,20 (d, J = 4,3Hz, 1H) ; de 7,31 à 7,69 (m, 8H) ; 7,97 (m, J = 8,6Hz, 2H) ; 8,41 (s, 1H) ; 8,84 (m, 1H). M+H = 349.

### Exemple 13: Enantiomère lévogyre du 1-{3-[2-(4-Chlorophényl)imidazo[1,2-a]pyridin-6-yl]phényl}éthanol (composé 61 du tableau)

En procédant comme décrit dans l'exemple 12, et à partir de 263 mg de 1-{3-[2-(4-Chlorophényl)imidazo[1,2-*a*]pyridin-6-yl]phényl}éthanol racémique, on obtient 112 mg de composé le plus retenu. F = 168 - 170°C. [α]_{D} = -13,6° (c = 0,41 ; MeOH). RMN 1H (DMSO-d6, δ en ppm) : 1,37 (d, J = 6,6Hz, 3H) ; 4,79 (m, 1H) ; 5,19 (d, J = 4,3Hz, 1H) ; de 7,31 à 7,69 (m, 8H) ; 7,97 (m, J = 8,7Hz, 2H) ; 8,41 (s, 1H) ; 8,84 (m,1H). M+H = 349.

### EXEMPLES REPRESENTATIFS

Dans ce tableau :
- la colonne « o/m/p » renseigne sur la position de substitution du groupe 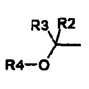 sur le noyau phényle « ortho/meta/para » ; Ph signifie Phényle ; C₅H₉ signifie cyclopentyle ; C₄H₈N signifie pyrrolidin-1-yle.
- la colonne « PF » renseigne les points de fusion des produits en degrés Celsius (°C) ou, lorsque les produits ont été isolés sous la forme de solide amorphe ou d'huile, ils sont caractérisés par leur masse [M+H];
- dans la colonne « Sel/base », « - » représente un composé sous forme de base libre, alors que « HCl » représente un composé sous forme de chlorhydrate et le rapport entre parenthèses est le rapport (acide:base).

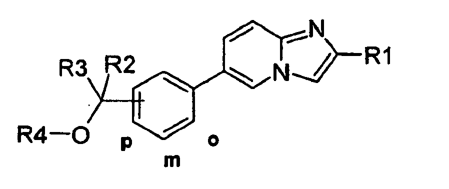

**Tableau 1**

| **N° Ex** | **R₁** | **o/m/p** | **R₂** | **R₃** | **R₄** | **Sel** | **PF ou [M+H]** |
|---|---|---|---|---|---|---|---|
| 1 | Ph | para | H | H | H | - | 238-240 |
| 2 | Ph | méta | H | H | H | - | 163-164 |
| **3** | 4-(C₄H₈N)Ph | para | H | H | H | - | 259-261 |
| **4** | 4-Phe-Ph | para | H | H | H | - | 310-312 |
| **5** | 3-CN-Ph | para | H | H | H | - | 205-207 |
| **6** | 3-CN-Ph | méta | H | H | H | - | 184-186 |
| **7** | 3-CN-Ph | méta | H | H | -(CH₂)₂OCH₃ | - | M+H=384 |
| **8** | 4-CN-Ph | méta | H | H | H | - | 199-201 |
| **9** | 2-Naphthyl | méta | H | H | H | - | 161-163 |
| **10** | 4-Me-Ph | méta | H | H | H | - | 171-173 |
| **11** | 4-(C₄H₈N)Ph | méta | H | H | H | - | 181-183 |
| **12** | 3-CF₃O-Ph | méta | H | H | H | - | 154-156 |
| **13** | 4-NO₂-Ph | méta | H | H | H | - | 199-201 |
| **14** | 4-N(C₂H₅)₂-Ph | para | H | H | H | - | 188-190 |
| **15** | 1-Naphthyl | méta | H | H | H | - | 306-308 |
| **16** | 2,4-Me₂-Ph | méta | H | H | H | - | 158-160 |
| **17** | 2-naphthyl | méta | H | H | -(CH₂)₂OCH₃ | - | 80-82 |
| **18** | 4-MeO-Ph | méta | H | H | H | - | 214-216 |
| **19** | 3-MeO-Ph | méta | H | H | H | - | 105-107 |
| **20** | 2-MeO-Ph | méta | H | H | H | - | 178-180 |
| **21** | 2,4-(MeO)₂-Ph | méta | H | H | H | - | 191-193 |
| **22** | 4-F-Ph | méta | H | H | H | - | 155-157 |
| **23** | 3,5-F₂-Phe | méta | H | H | H | - | 166-168 |
| **24** | 3-F-5-CF₃-Ph | méta | H | H | H | - | 178-179 |
| **25** | 4-Cl-Ph | méta | H | H | H | - | 181-182 |
| **26** | 4-Cl-Ph | méta | H | H | H | HCl (1:1) | 244-246 |
| **27** | 3-Cl-Ph | méta | H | H | H | - | 260-262 |
| **28** | 4-Cl-3-Me-Ph | méta | H | H | H | - | 157-159 |
| **29** | 3,4-Cl₂-Ph | méta | H | H | H | - | 187-189 |
| **30** | 3-F-Ph | méta | H | H | H | - | 125-127 |
| **31** | 4-Me-Ph | méta | H | H | 4-Cl-Ph | - | 182-184 |
| **32** | 4-Me-Ph | méta | H | H | 4-(CH₃CONH)Ph | - | 191-193 |
| **33** | 4-Me-Ph | méta | H | H | 3-NO₂-Ph | - | 78-80 |
| **34** | 4-Me-Ph | méta | H | H | 4-Me-Ph | - | 141-143 |
| **35** | 4-Me-Ph | méta | H | H | 4-CN-Ph | | 193-195 |
| **36** | 4-Me-Ph | méta | H | H | 4-(CH₃CO)Ph | - | 186-188 |
| **37** | 4-Me-Ph | méta | H | H | 4-CF₃-Ph | - | 173-175 |
| **38** | 3-F-4-Me-Ph | méta | H | H | H | - | 171-173 |
| **39** | 4-Cl-Ph | méta | Me | Me | H | - | 166-168 |
| **40** | 4-Cl-Ph | ortho | H | H | H | - | 164-166 |
| **41** | 4-Cl-Ph | ortho | H | H | H | HCl (1:1) | 238-239 |
| **42** | 4-Cl-Ph | méta | H | Me | H | - | 174-176 |
| **43** | 2,4-Cl₂-Ph | méta | H | H | H | - | 190-192 |
| **44** | 2,4-F₂-Ph | méta | H | H | H | - | 167-169 |
| **45** | 3,4-F₂-Ph | méta | H | H | H | - | 179-181 |
| **46** | 3,4-F₂-Ph | méta | H | H | NCl | H (1:1) | 243 - 246 |
| **47** | 2-Cl-Ph | méta | H | H | H | - | 72-174 |
| **48** | 4-CF₃-Ph | méta | H | H | H | - | 165-167 |
| **49** | 4-CHF₂-Ph | méta | H | H | H | - | 153-155 |
| **50** | 4-Cl-Ph | ortho | H | Me | H | - | 216-218 |
| **51** | 4-Cl-Ph | ortho | H | H | Me | - | 106-108 |
| **52** | 4-Cl-Ph | ortho | H | H | Me | HCl (1:1) | 228-230 |
| **53** | 4-Cl-Ph | para | H | H | Me | - | 104-106 |
| **54** | 4-Cl-Ph | méta | H | C₂H₅ | H | - | 153-155 |
| **55** | 4-Cl-Ph | méta | H | C₄H₉ | H | - | 159-161 |
| **56** | 4-Cl-Ph | méta | H | C₆H₁₃ | H | - | 140-142 |
| **57** | 4-Cl-Ph | méta | H | iBu | H | - | 166-168 |
| **58** | 4-Cl-Ph | méta | H | C₅H₉ | H | - | M+H = 403 |
| **59** | 4-Cl-Ph | méta | H | Ph | H | - | 242-244 |
| **60** | 4-Cl-Ph | méta | H | Me | H | - | 168-170 |
| **61** | 4-Cl-Ph | méta | H | Me | H | - | 168-170 |
| **62** | 4-Cl-Ph | méta | H | H | Me | - | 135-137 |
| **63** | 4-Cl-Ph | para | H | Me | H | - | 180-182 |
| **64** | 2,4-F₂-Ph | méta | H | Me | H | - | 179-181 |
| **65** | 4-F-Ph | méta | H | Me | H | - | 170-172 |
| **66** | 3,4-F₂-Ph | méta | H | Me | H | - | 196-198 |
| **67** | 2-Cl-Ph | méta | H | Me | H | - | 156-158 |
| **68** | 3-F-Ph | méta | H | Me | H | - | 166-168 |
| **69** | 3-Cl-Ph | méta | H | Me | H | - | 154-156 |
| **70** | 4-OH-Ph | méta | H | H | H | - | 255,2 - 256,7 |

Les produits 60 et 61 sont les énantiomères du produit racémique 42.

Les composés selon l'invention ont fait l'objet d'essais pharmacologiques permettant de déterminer leur effet modulateur sur Nurr-1/NOT.

### Evaluation de l'activité in vitro sur cellule N2A

Des essais ont consisté à mesurer l'activité *in vitro* des composés de l'invention sur une lignée cellulaire (N2A) exprimant de manière endogène le récepteur de souris Nurr1 et transfectée de manière stable avec l'élément de réponse liant NOT (NBRE) couplé au gène rapporteur luciférase. Les EC₅₀ sont comprises entre 0,01 et 1000 nM. Les essais ont été réalisés selon le mode opératoire décrit ci-dessous.

La lignée cellulaire Neuro-2A provient de source commerciale standard (ATCC). Le clone Neuro-2A a été obtenu à partir d'une tumeur spontanée provenant d'une souche de souris A albino par R.J Klebe et col. Cette lignée Neuro-2A est ensuite stablement transfectée avec 8NBRE-luciférase. Les cellules N2A-8NBRE sont cultivées jusqu'à confluence dans des flacons de culture de 75 cm² contenant du DMEM supplémenté par 10% de sérum foetal de veau, 4.5 g/L de glucose et 0.4 mg/ml de Généticine. Après une semaine de culture les cellules sont récupérées par de la trypsine 0.25% pendant 30 secondes puis remises en suspension dans du DMEM sans rouge de phénol contenant 4.5g/L de glucose, 10% de sérum délipidé Hyclone et déposées dans des plaques blanches 96 puits fond transparent. Les cellules sont déposées à raison de 60.000 par puit dans 75 µL pendant 24 heures avant l'addition des produits. Les produits sont appliqués dans 25µL et incubés 24 heures supplémentaires. Le jour de la mesure, on ajoute à chaque puit un volume équivalent (100µL) de Steadylite, puis attend 30 minutes pour obtenir une lyse complète des cellules et la production maximale du signal. Les plaques sont ensuite mesurées dans un compteur de luminescence pour microplaques après avoir été scellées par un film adhésif. Les produits sont préparés sous forme de solution stock à 10⁻²M, puis dilués dans 100% de DMSO. Chaque concentration de produit est préalablement diluée dans du milieu de culture avant incubation avec les cellules contenant ainsi 0.625% final de DMSO.

Par exemple, les composés n° 17, 31, 39 et 40 du tableau ont montré une EC₅₀ de respectivement 3,6 nM, 14 nM, 0,7 nM et 0,7 nM.

### Evaluation de la liaison au récepteur humain NOT

La liaison directe entre des composés de l'invention et le récepteur humain NOT a été évaluée en utilisant la technologie SPR (surface plasmon resonance). Dans cet essai la protéine est immobilisée de façon covalente à la matrice et la molécule à étudier est injectée dans la chambre contenant la sensor chip (biocapteur ou surface réactive). Le signal est directement proportionnel à la quantité de produit fixé à la protéine. Les essais de liaison ont été réalisés dans un instrument BIACORE S51 (Biacore Inc., Piscataway N.J.). La protéine entière GST-NOT (NOT-FL) a été fournie par Invitrogen (PV3265).

Le domaine de liaison au ligand de NOT (His-Thr-NOT 329-598) a été exprimé et purifié comme décrit dans Nature 423, 555-560. Les deux protéines, diluées à une concentration de 20 µg/ml dans un tampon acétate pH 5.0 contenant 5 mM de DTT, ont été immobilisées sur une surface de carboxymethyl 5' dextrane (CM5 sensor chip, Biacore Inc.) par couplage amine en suivant le protocole recommandé par Biacore en éluant par un tampon HBS-N (10 mM HEPES, 0.15 M NaCl, 3 mM EDTA, pH 7.4). Approximativement 10000-15000 unités de resonance (RU) des protéines sont capturées sur la surface du sensor chip CM5. Les solutions stock des composés à étudier à 1,5 mM dans le DMSO sont diluées en série dans du tampon d'élution (50 mM HEPES pH8; 150 mM NaCl; 10 mM MgCl₂; 2% DMSO, 1 mM DTT) à des concentrations allant de 3,75 à 0,1 µM. Chaque concentration de produit est injectée à 4°C pendant 1 minute à 30 µl/min. La dissociation a été enregistrée pendant 5 minutes sans autre procédure de régénération de la surface. Les signaux obtenus sont corrigés en testant chaque concentration de produit sur une surface de dextrane non modifiée (blanc). Le signal dû au tampon de migration est déduit du signal total (« double referencing ») ainsi que l'effet du DMSO. L'analyse des signaux est effectuée à l'aide du logiciel d'analyse Biacore S51 (version 1.2.1). Les composés sont ensuite classés en fonction de leur niveau de fixation maximal et de paramètres cinétiques de liaison à la protéine immobilisée.

A titre d'exemple, le composé n° 9 a une affinité forte et le composé n° 10 a une affinité moyenne.

Il apparaît donc que les composés selon l'invention ont un effet modulateur de NOT.

Les composés selon l'invention peuvent donc être utilisés pour la préparation de médicaments pour leur application en thérapeutique dans le traitement ou la prévention de maladies impliquant les récepteurs NOT.

Ainsi, selon un autre de ses aspects, l'invention a pour objet des médicaments qui comprennent un composé de formule (I), ou un sel d'addition de ce dernier à un acide pharmaceutiquement acceptable.

Ces médicaments trouvent leur emploi en thérapeutique, notamment dans le traitement et la prévention des maladies neurodégénératives telles que par exemple la maladie de Parkinson, d'Alzheimer, les tauopathies (ex. la paralysie progressive supranucléaire, la démence fronto temporale, la dégénérescence corticobasale, la maladie de Pick), la sclérose en plaque ; les traumatismes cérébraux comme l'ischémie et les traumatismes crâniens et l'épilepsie; les maladies psychiatriques comme la schizophrénie, la dépression, la dépendance à une substance, les troubles du déficit de l'attention et de l'hyperactivité ; les maladies inflammatoires comme les pathologies vasculaires, l'athérosclérose, les inflammations des articulations, l'arthrose, l'arthrite rhumatoïde ostéoarthrite, maladies inflammatoires allergiques telle que l'asthme et pour finir le traitement de l'ostéoporose, les cancers.

Ces composés pourraient être aussi utilisés comme traitement associé à des greffes et/ou transplantations de cellules souches.

Selon un autre de ses aspects, la présente invention concerne des compositions pharmaceutiques comprenant, en tant que principe actif, un composé selon l'invention. Ces compositions pharmaceutiques contiennent une dose efficace d'au moins un composé selon l'invention, ou un sel pharmaceutiquement acceptable dudit composé, ainsi qu'au moins un excipient pharmaceutiquement acceptable.

Lesdits excipients sont choisis selon la forme pharmaceutique et le mode d'administration souhaité, parmi les excipients habituels qui sont connus de l'Homme du métier.

Dans les compositions pharmaceutiques de la présente invention pour l'administration orale, sublinguale, sous-cutanée, intramusculaire, intra-veineuse, topique, locale, intratrachéale, intranasale, transdermique ou rectale, le principe actif de formule (I) ci-dessus, ou son sel, peut être administré sous forme unitaire d'administration, en mélange avec des excipients pharmaceutiques classiques, aux animaux et aux êtres humains pour la prophylaxie ou le traitement des troubles ou des maladies ci-dessus.

Les formes unitaires d'administration appropriées comprennent les formes par voie orale telles que les comprimés, les gélules molles ou dures, les poudres, les granules et les solutions ou suspensions orales, les formes d'administration sublinguale, buccale, intratrachéale, intraoculaire, intranasale, par inhalation, les formes d'administration topique, transdermique, sous-cutanée, intramusculaire ou intraveineuse, les formes d'administration rectale et les implants. Pour l'application topique, on peut utiliser les composés selon l'invention dans des crèmes, gels, pommades ou lotions.

A titre d'exemple, une forme unitaire d'administration d'un composé selon l'invention sous forme de comprimé peut comprendre les composants suivants :

| | |
|---|---|
| Composé selon l'invention | 50,0 mg |
| Mannitol | 223,75 mg |
| Croscarmellose sodique | 6,0 mg |
| Amidon de maïs | 15,0 mg |
| Hydroxypropyl-méthylcellulose | 2,25 mg |
| Stéarate de magnésium | 3,0 mg |

Il peut y avoir des cas particuliers où des dosages plus élevés ou plus faibles sont appropriés ; de tels dosages ne sortent pas du cadre de l'invention. Selon la pratique habituelle, le dosage approprié à chaque patient est déterminé par le médecin selon le mode d'administration, le poids et la réponse dudit patient.

La présente invention, selon un autre de ses aspects, concerne également une méthode de traitement des pathologies ci-dessus indiquées qui comprend l'administration, à un patient, d'une dose efficace d'un composé selon l'invention, ou un de ses sels pharmaceutiquement acceptables.

## Revendications

1. Composé répondant à la formule (I) : 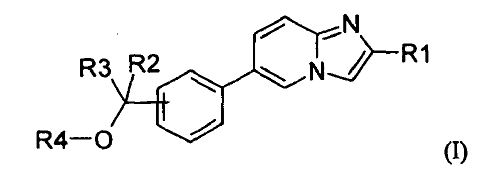 dans laquelle :
R₁ représente :
un groupe phényle ou un groupe naphthyle, ces deux groupes pouvant éventuellement être substitués par un ou plusieurs atomes ou groupes choisis indépendamment les uns des autres parmi les atomes ou groupes suivants : halogène, (C₁-C₆)alkyle, (C₃-C₇)cycloalkyle, (C₃-C₇)cycloalkyl(C₁-C₃)alkylène, halo(C₁-C₆)alkyle, (C₁-C₆)alcoxy, (C₃-C₇)cycloalcoxy, (C₃-C₇)cycloalkyl(C₁-C₃)alkylène-oxy, halo(C₁-C₆)alcoxy, (C₁-C₆)thioalkyle, -S(O)(C₁-C₆)alkyle, -S(O)₂(C₁-C₆-alkyle), hydroxyle, cyano, nitro, hydroxy(C₁-C₆)alkylène, NRaRb(C₁-C₆)alkylène, (C₁-C₆)alcoxy(C₁-C₆)alkylène-oxy, NRaRb, CONRaRb, SO₂NRaRb, NRcCORd, OC(O)NRaRb, NRcC(O)ORe, NRcSO₂Re, aryl(C₁-C₆)alkylène, aryle ou hétéroaryle, l'aryle ou l'hétéroaryle étant éventuellement substitué par un ou plusieurs substituants choisis parmi un halogène, un groupe (C₁-C₆)alkyle, (C₃-C₇)cycloalkyle, (C₃-C₇)cycloalkyl-(C₁-C₃)-alkylène, halo(C₁-C₆)alkyle, (C₁-C₆)alcoxy, halo(C₁-C₆)alcoxy, NRaRb, hydroxyle, nitro ou cyano ;
R₂ et R₃ représentent, indépendamment l'un de l'autre,
un atome d'hydrogène,
un groupe (C₁-C₆)alkyle, (C₃-C₇)cycloalkyle ou (C₃-C₇)cycloalkyl(C₁-C₃)alkylène, ce groupe étant éventuellement substitué par un groupe Rf ;
un groupe aryle, l'aryle étant éventuellement substitué par un ou plusieurs substituants choisis parmi un halogène, un groupe (C₁-C₆)alkyle, (C₃-C₇)cycloalkyle, (C₃-C₇)cycloalkyl(C₁-C₃)alkylène, halo(C₁-C₆)alkyle, (C₁-C₆)alcoxy, halo(C₁-C₆)alcoxy, NRaRb, hydroxyle, nitro ou cyano ;
R₄ représente :
un atome d'hydrogène,
un groupe (C₁-C₆)alkyle, (C₃-C₇)cycloalkyle ou (C₃-C₇)cycloalkyl(C₁-C₃)alkylène, ce groupe étant éventuellement substitué par un groupe Rf ;
un groupe aryle, l'aryle étant éventuellement substitué par un ou plusieurs substituants choisis parmi un halogène, un groupe (C₁-C₆)alkyle, (C₃-C₇)cycloalkyle, (C₃-C₇)cycloalkyl(C₁-C₃)alkyléne, halo(C₁-C₆)alkyle, (C₁-C₆)alcoxy, (C₃-C₇)cycloalcoxy, (C₃-C₇)cycloalkyl(C₁-C₃)alkylène-oxy, halo(C₁-C₆)alcoxy, NRaRb, hydroxyle, nitro, cyano, (C₁-C₆)alkyl(CO)-, CONRaRb, NRcCORd, OC(O)NRaRb, NRcC(O)ORe ou aryle, l'aryle étant éventuellement substitué par un ou plusieurs substituants choisis parmi un halogène, un groupe (C₁-C₆)alkyle, (C₃-C₇)cycloalkyle, (C₃-C₇)cycloalkyl(C₁-C₃)alkylène, halo(C₁-C₆)alkyle, (C₁-C₆)alcoxy, halo(C₁-C₆)alcoxy, NRaRb, hydroxyle, nitro ou cyano ;
Ra et Rb représentent, indépendamment l'un de l'autre,
un atome d'hydrogène ou un groupe (C₁-C₆)alkyle, (C₃-C₇)cycloalkyle, (C₃-C₇)cycloalkyl(C₁-C₃)alkylène, aryl(C₁-C₆)alkylène ou aryle ;
ou Ra et Rb forment ensemble, avec l'atome d'azote qui les porte, un groupe azétidine, pyrrolidine, pipéridine, azépine, morpholine, thiomorpholine, pipérazine, homopipérazine, ce groupe étant éventuellement substitué par un groupe (C₁-C₆)alkyle, (C₃-C₇)cycloalkyle, (C₃-C₇)cycloalkyl(C₁-C₆)alkylène, aryle ou aryl(C₁-C₆)alkylène ;
Rc et Rd représentent, indépendamment l'un de l'autre,
un atome d'hydrogène ou un groupe (C₁-C₆)alkyle, (C₃-C₇)cycloalkyle, (C₃-C₇)cycloalkyl(C₁-C₃)alkylène, aryl(C₁-C₆)alkylène, aryle ;
ou Rc et Rd forment ensemble un groupe (C₂-C₅)alkylène ;
Re représente un groupe (C₁-C₆)alkyle, (C₃-C₇)cyloalkyle, (C₃-C₇)cycloalkyl(C₁-C₃)alkylène, aryl(C₁-C₆)alkylène, aryle ;
ou Rc et Re forment ensemble un groupe (C₂-C₅)alkylène ;
Rf représente un atome d'halogène, un groupe (C₁-C₆)alcoxy, halo(C₁-C₆)alcoxy, (C₃-C₇)cycloalcoxy, (C₃-C₇)cycloalkyl(C₁-C₆)alkylène-oxy, hydroxyle, cyano, NRaRb, C(O)NRaRb, NRcCORd, OC(O)NRaRb, NRcCOORe, SO₂NRaRb, NRcSO₂Re, aryl(C₁-C₆)alkylène, aryle, l'aryle étant éventuellement substitué par un
ou plusieurs substituants choisis parmi un halogène, un groupe (C₁-C₆)alkyle, (C₃-C₇)cycloalkyle, (C₃-C₇)cycloalkyl(C₁-C₃)alkylène, halo(C₁-C₆)alkyle, (C₁-C₆)alcoxy, halo(C₁-C₆)alcoxy, NRaRb, hydroxyle, nitro ou cyano ;
à l'état de base ou de sel d'addition à un acide.

2. Composé de formule (I) selon la revendication 1, **caractérisé en ce que** :
R₁ représente un groupe naphthyle ou un groupe phényle pouvant éventuellement être substitué par un ou plusieurs atomes ou groupes choisis indépendamment les uns des autres parmi les atomes ou groupes suivants : halogène, (C₁-C₆)alkyle, (C₃-C₇)cycloalkyle, (C₃-C₇)cycloalkyl(C₁-C₆)alkylène, (C₁-C₆)alcoxy, (C₃-C₇)cycloalkyl(C₁-C₆)alcoxy, hydroxy, cyano, nitro, NRaRb, hydroxy(C₁-C₆)alkyle, (C₁-C₆)alcoxy(C₁-C₆)alkyle, halo(C₁-C₆)alkyle, halo(C₁-C₆)alcoxy ou aryle, l'aryle étant éventuellement substitué par un ou plusieurs substituants choisi parmi un halogène, un groupe (C₁-C₆)alkyle, (C₃-C₇)cycloalkyle, (C₃-C₇)cycloalkyl-(C₁-C₃)-alkyléne, halo(C₁-C₆)alkyle, (C₁-C₆)alcoxy, halo(C₁-C₆)alcoxy, NRaRb, hydroxyle, nitro ou cyano ;
à l'état de base ou de sel d'addition à un acide.

3. Composé de formule (I) selon la revendication 1 ou 2, **caractérisé en ce que** :
R₂, R₃ représentent, indépendamment l'un de l'autre, un atome d'hydrogène,
un groupe (C₁-C₆)alkyle, (C₃-C₇)cycloalkyle ou (C₃-C₇)cycloalkyl(C₁-C₃)alkylène éventuellement substitué par un groupe Rf ;
R₄ représente : un atome d'hydrogène,
un groupe (C₁-C₆)alkyle, (C₃-C₇)cycloalkyle ou (C₃-C₇)cycloalkyl(C₁-C₃)alkylène éventuellement substitué par un groupe Rf,
un groupe aryle éventuellement substitué par un ou plusieurs substituants choisis parmi un halogène, un groupe (C₁-C₆)alkyle, (C₃-C₇)cycloalkyle, (C₃-C₇)cycloalkyl(C₁-C₃)alkyléne, halo(C₁-C₆)alkyle, (C₁-C₆)alcoxy, (C₃-C₇)cycloalcoxy, (C₃-C₇)cycloalkyl(C₁-C₃)alkyléne-oxy, halo(C₁-C₆)alcoxy, NRaRb, hydroxyle, nitro, cyano , (C₁-C₆)alkyl(CO)-, NRcCORd, ou aryle, l'aryle étant éventuellement substitué par un ou plusieurs substituants choisi parmi un halogène, un groupe (C₁-C₆)alkyle, (C₃-C₇)cycloalkyle, (C₃-C₇)cycloalkyl(C₁-C₃)alkylène, halo(C₁-C₆)alkyle, (C₁-C₆)alcoxy, halo(C₁-C₆)alcoxy, NRaRb, hydroxyle, nitro ou cyano ;
à l'état de base ou de sel d'addition à un acide.

4. Composé de formule (I) selon l'une quelconque des revendications 1 à 3, **caractérisé en ce que** :
le substituant 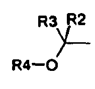 est en position méta sur le phényle ; à l'état de base ou de sel d'addition à un acide.

5. Composé de formule (I) selon la revendication 1 ou 3, **caractérisé en ce que**
R₄ représente un atome d'hydrogène et
R₂ et R₃ représentent, indépendamment l'un de l'autre, un atome hydrogène, un groupe (C₁-C₆)alkyle, (C₃-C₇)cycloalkyle ou (C₃-C₇)cycloalkyl(C₁-C₃)alkylène éventuellement substitué par un groupe Rf;
à l'état de base ou de sel d'addition à un acide.

6. Composé de formule (I) selon la revendication 1, **caractérisé en ce que** R₁ représente un groupe naphthyle ou un groupe phényle pouvant éventuellement être substitué par un ou plusieurs atomes ou groupes choisis indépendamment les uns des autres parmi les atomes ou groupes suivants : halogène, (C₁-C₆)alkyle, (C₃-C₇)cycloalkyle, (C₃-C₇)cycloalkyl(C₁-C₆)alkylène, (C₁-C₆)alcoxy, (C₃-C₇)cycloalkyl(C₁-C₆)alcoxy, hydroxy, cyano, nitro, NRaRb, hydroxy(C₁-C₆)alkyle, (C₁-C₆)alcoxy(C₁-C₆)alkyle, halo(C₁-C₆)alkyle, halo(C₁-C₆)alcoxy ou aryle, l'aryle étant éventuellement substitué par un ou plusieurs substituants choisis parmi un halogène, un groupe (C₁-C₆)alkyle, (C₃-C₇)cycloalkyle, (C₃-C₇)cycloalkyl-(C₁-C₃)-alkylène, halo(C₁-C₆)alkyle, (C₁-C₆)alcoxy, halo(C₁-C₆)alcoxy, NRaRb, hydroxyle, nitro ou cyano, à l'état de base ou de sel d'addition à un acide ;
R₂, R₃ représentent, indépendamment l'un de l'autre,
un atome d'hydrogène,
un groupe (C₁-C₆)alkyle, (C₃-C₇)cycloalkyle ou (C₃-C₇)cycloalkyl(C₁-C₃)alkylène, éventuellement substitué par un groupe Rf ;
un groupe aryle, l'aryle étant éventuellement substitué par un ou plusieurs substituants choisis parmi un halogène, un groupe (C₁-C₆)alkyle, (C₃-C₇)cycloalkyle, (C₃-C₇)cycloalkyl(C₁-C₃)alkylène, halo(C₁-C₆)alkyle, (C₁-C₆)alcoxy, halo(C₁-C₆)alcoxy, NRaRb, hydroxyle, nitro ou cyano ;
R₄ représente :
un atome d'hydrogène,
un groupe (C₁-C₆)alkyle, (C₃-C₇)cycloalkyle ou (C₃-C₇)cycloalkyl(C₁-C₃)alkylène, éventuellement substitué par un groupe Rf ,
un groupe aryle, l'aryle étant éventuellement substitué par un ou plusieurs substituants choisis parmi un halogène, un groupe (C₁-C₆)alkyle, (C₃-C₇)cycloalkyle, (C₃-C₇)cycloalkyl(C₁-C₃)alkylène, halo(C₁-C₆)alkyle, nitro, cyano, (C₁-C₆)alkyl(CO)-, NRcCORd ;
à l'état de base ou de sel d'addition à un acide.

7. Composé de formule (I) selon la revendication 1, **caractérisé en ce que**
R₁ représente un groupe naphthyle ou un groupe phényle pouvant éventuellement être substitué par un ou plusieurs atomes ou groupes choisis indépendamment les uns des autres parmi les atomes ou groupes suivants : halogène, (C₁-C₆)alkyle, (C₃-C₇)cycloalkyle, (C₃-C₇)cycloalkyl(C₁-C₆)alkylène, (C₁-C₆)alcoxy, (C₃-C₇)cycloalkyl(C₁-C₆)alcoxy, hydroxy, cyano, nitro, NRaRb, hydroxy(C₁-C₆)alkyle, (C₁-C₆)alcoxy(C₁-C₆)alkyle, halo(C₁-C₆)alkyle, halo(C₁-C₆)alcoxy,
R₂ et R₃ représentent, indépendamment l'un de l'autre,
un atome d'hydrogène,
un groupe (C₁-C₆)alkyle, (C₃-C₇)cycloalkyle ou (C₃-C₇)cycloalkyl(C₁-C₃)alkylène, ce groupe étant éventuellement substitué par un groupe Rf ;
un groupe aryle, l'aryle étant éventuellement substitué par un ou plusieurs substituants choisis parmi un halogène, un groupe (C₁-C₆)alkyle, (C₃-C₇)cycloalkyle, (C₃-C₇)cycloalkyl(C₁-C₃)alkylène, halo(C₁-C₆)alkyle, (C₁-C₆)alcoxy, halo(C₁-C₆)alcoxy, NRaRb, hydroxyle, nitro ou cyano ;
R₄ représente : un atome d'hydrogène,
un groupe (C₁-C₆)alkyle, (C₃-C₇)cycloalkyle ou (C₃-C₇)cycloalkyl(C₁-C₃)alkylène, ce groupe étant éventuellement substitué par un groupe Rf ,
un groupe aryle, l'aryle étant éventuellement substitué par un ou plusieurs substituants choisis parmi un halogène, un groupe (C₁-C₆)alkyle, (C₃-C₇)cycloalkyle, (C₃-C₇)cycloalkyl(C₁-C₃)alkylène, halo(C₁-C₆)alkyle, nitro, cyano, (C₁-C₆)alkyl(CO)-, NRcCORd
à l'état de base ou de sel d'addition à un acide.

8. Composés de formule (I) selon la revendication 1, choisis parmi les composés suivants :
[4-(2-Phénylimidazo[1,2*-a*]pyridin-6-yl)phényl]méthanol
[3-(2-Phénylimidazo[1,2*-a*]pyridin-6-yl)phényl]méthanol
{4-[2-(4-Pyrrolidin-1-ylphényl)-imidazo[1,2*-a*]pyridin-6-yl]phényl}méthanol
[4-(2-Biphényl-4-ylimidazo[1,2*-a*]pyridin-6-yl)phényl]méthanol
3-[6-(4-Hydroxyméthylphényl)imidazo[1,2*-a*]pyridin-2-yl]benzonitrile
3-[6-(3-Hydroxyméthylphényl)imidazo[1,2*-a*]pyridin-2-yl]benzonitrile
3-{6-[3-(2-Méthoxyéthoxyméthyl)phényl]imidazo[1,2*-a*]pyridin-2-yl}benzonitrile
4-[6-(3-Hydroxyméthylphényl)imidazo[1,2*-a*]pyridin-2-yl]benzonitrile
[3-[2-(Naphthalèn-2-yl)imidazo[1,2*-a*]pyridin-6-yl]phényl]méthanol
[3-(2*-p*-Tolylimidazo[1,2*-a*]pyridin-6-yl)phényl]méthanol
{3-[2-(4-Pyrrolidin-1-ylphényl)imidazo[1,2*-a*]pyridin-6-yl]phényl}méthanol
{3-[2-(3-Trifluorométhoxyphényl)imidazo[1,2*-a*]pyridin-6-yl]phényl}méthanol
{3-[2-(4-Nitrophényl)imidazo[1,2*-a*]pyridin-6-yl]phényl} méthanol
{4-[2-(4-Diéthylaminophényl)imidazo[1,2*-a*]pyridin-6-yl]phényl} méthanol
3-(2-Naphthalèn-1-ylimidazo[1,2*-a*]pyridin-6-yl)phényl]méthanol
{3-[2-(2,4-Diméthylphényl)imidazo[1,2*-a*]pyridin-6-yl]phényl} méthanol
6-[3-(2-Méthoxyéthoxyméthyl)phényl]-2-naphthalèn-2-ylimidazo[1,2*-a*]pyridine
{3-[2-(4-Méthoxyphényl)imidazo[1,2*-a*]pyridin-6-yl]phényl}méthanol
{3-[2-(3-Méthoxyphényl)imidazo[1,2*-a*]pyridin-6-yl]phényl} méthanol
{3-[2-(2-Méthoxyphényl)imidazo[1,2*-a*]pyridin-6-yl]phényl}méthanol
{3-[2-(2,4-Diméthoxyphényl)imidazo[1,2*-a*]pyridin-6-yl]phényl} méthanol
{3-[2-(4-Fluorophényl)imidazo[1,2*-a*]pyridin-6-yl]phényl}méthanol
{3-[2-(3,5-Difluorophényl)imidazo[1,2*-a*]pyridin-6-yl]phényl} méthanol
{3-[2-(3-Fluoro-5-trifluorométhylphényl)imidazo[1,2*-a*]pyridin-6-yl]phényl} méthanol
{3-[2-(4-Chlorophényl)imidazo[1,2*-a*]pyridin-6-yl]phényl} méthanol
Chlorhydrate (1:1) de {3-[2-(4-Chlorophényl)imidazo[1,2*-a*]pyridin-6-yl]phényl}méthanol
{3-[2-(3-Chlorophényl)imidazo[1,2*-a*]pyridin-6-yl]phényl}méthanol
{3-[2-(4-Chloro-3-méthylphényl)imidazo[1,2*-a*]pyridin-6-yl]phényl} méthanol
{3-[2-(3,4-Dichlorophényl)imidazo[1,2*-a*]pyridin-6-yl]phényl}méthanol
{3-[2-(3-Fluorophényl)imidazo[1,2*-a*]pyridin-6-yl]phényl}méthanol
6-[3-(4-Chlorophénoxyméthyl)phényl]-2*-p*-tolylimidazo[1,2*-a*]pyridine
*N*-{4-[3-(2*-p*-Tolylimidazo[1,2*-a*]pyridin-6-yl)benzyloxy]phényl}acétamide
6-[3-(3-Nitrophénoxyméthyl)phényl]-2*-p*-tolylimidazo[1,2*-a*]pyridinc
6-[3-(4-Méthylphénoxyméthyl)phényl]-2*-p*-tolylimidazo[1,2*-a*]pyridine
4-[3-(2*-p*-Tolylimidazo[1,2*-a*]pyridin-6-yl)benzyloxy]benzonitrile
1-{4-[3-(2*-p*-Tolylimidazo[1,2*-a*]pyridin-6-yl)benzyloxy]phényl}éthanone
6-[3-(4-Trifluorométhylphénoxyméthyl)phényl]-2*-p*-tolylimidazo[1,2-a]pyridine
{3-[2-(3-Fluoro-4-méthylphényl)imidazo[1,2*-a*]pyridin-6-yl]phényl} méthanol
2-{3-[2-(4-Chlorophényl)imidazo[1,2*-a*]pyridin-6-yl]phényl} propan-2-ol
{2-[2-(4-Chlorophényl)imidazo[1,2*-a*]pyridin-6-yl]phényl}méthanol
Chlohydrate (1:1) de {2-[2-(4-Chlorophényl)imidazo[1,2*-a*]pyridin-6-yl]phényl}méthanol
1-{3-[2-(4-Chlorophényl)imidazo[1,2*-a*]pyridin-6-yl]phényl}éthanol racémique
{3-[2-(2,4-Dichlorophényl)imidazo[1,2*-a*]pyridin-6-yl]phényl}méthanol
{3-[2-(2,4-Difluorophényl)imidazo[1,2*-a*]pyridin-6-yl]phényl}méthanol
{3-[2-(3,4-Difluorophényl)imidazo[1,2*-a*]pyridin-6-yl]phényl}méthanol
Chlohydrate (1:1) de {3-[2-(3,4-Difluorophényl)imidazo[1,2*-a*]pyridin-6-yl]phényl}méthanol
{3-[2-(2-Chlorophényl)imidazo[1,2*-a*]pyridin-6-yl]phényl}méthanol
{3-[2-(4-Trifluorométhylphényl)imidazo[1,2*-a*]pyridin-6-yl]phényl} méthanol
{3-[2-(4-(Difluorométhyl)phényl)imidazo[1,2*-a*]pyridin-6-yl]phényl}méthanol
1-{2-[2-(4-Chlorophényl)imidazo[1,2*-a*]pyridin-6-yl]phényl}éthanol racémique
2-(4-Chlorophényl)-6-(2-méthoxyméthylphényl)imidazo[1,2*-a*] pyridine
Chlohydrate (1:1) de 2-(4-Chlorophényl)-6-(2-méthoxyméthylphényl)imidazo[1,2*-a*]pyridine
2-(4-Chlorophényl)-6-(4-méthoxyméthylphényl)imidazo[1,2*-a*]pyridine
1-{3-[2-(4-Chlorophényl)imidazo[1,2*-a*]pyridin-6-yl]phényl}propan-1-ol racémique
1-{3-[2-(4-Chlorophényl)imidazo[1,2*-a*]pyridin-6-yl]phényl}pentan-1-ol racémique
1-{3-[2-(4-Chlorophényl)imidazo[1,2*-a*]pyridin-6-yl]phényl}heptan-1-ol racémique
1-{3-[2-(4-Chlorophényl)imidazo[1,2*-a*]pyridin-6-yl]phényl}-3-méthylbutan-1-ol racémique
{3-[2-(4-Chlorophényl)imidazo[1,2*-a*]pyridin-6-yl]phényl}cyclopentylméthanol racémique
{3-[2-(4-Chlorophényl)imidazo[1,2*-a*]pyridin-6-yl]phényl}phenylméthanol racémique
Enantiomère dextrogyre de 1-{3-[2-(4-Chlorophényl)imidazo[1,2*-a*]pyridin-6-yl]phényl}éthanol
Enantiomère lévogyre de 1-{3-[2-(4-Chlorophényl)imidazo[1,2*-a*]pyridin-6-yl]phényl}éthanol
2-(4-Chlorophényl)-6-(3-méthoxyméthylphényl)imidazo[1,2*-a*]pyridine
1-{4-[2-(4-Chlorophényl)imidazo[1,2*-a*]pyridin-6-yl]phényl}éthanol racémique
1- {3-[2-(2,4-Difluorophényl)imidazo[1,2*-a*]pyridin-6-yl]phényl}éthanol racémique
1-{3-[2-(4-Fluororophényl)imidazo[1,2*-a*]pyridin-6-yl]phényl}éthanol racémique
1-{3-[2-(3,4-Difluorophényl)imidazo[1,2*-a*]pyridin-6-yl]phényl}éthanol racémique
1-{3-[2-(2-Chlorophényl)imidazo[1,2*-a*]pyridin-6-yl]phényl}éthanol racémique
1-{3-[2-(3-Fluorophényl)imidazo[1,2*-a*]pyridin-6-yl]phényl}éthanol racémique
1-{3-[2-(3-Chlorophényl)imidazo[1,2*-a*]pyridin-6-yl]phényl}éthanol racémique
4-[6-(3-Hydroxyméthylphényl)imidazo[1,2*-a*]pyridin-2-yl]phénol
à l'état de base ou de sel d'addition à un acide.

9. Médicament, **caractérisé en ce qu'**il comprend un composé de formule (I) selon l'une quelconque des revendications 1 à 8, ou un sel d'addition de ce composé à un acide pharmaceutiquement acceptable.

10. Composition pharmaceutique, **caractérisée en ce qu'**elle comprend un composé de formule (I) selon l'une quelconque des revendications 1 à 8, ou un sel pharmaceutiquement acceptable, de ce composé, ainsi qu'au moins un excipient pharmaceutiquement acceptable.

11. Utilisation d'un composé de formule (I) selon l'une quelconque des revendications 1 à 8 pour la préparation d'un médicament destiné au traitement et à la prévention des maladies neurodégénératives.

12. Utilisation d'un composé de formule (I) selon l'une quelconque des revendications 1 à 8 pour la préparation d'un médicament destiné au traitement et à la prévention des traumatismes cérébraux et de l'épilepsie.

13. Utilisation d'un composé de formule (I) selon l'une quelconque des revendications 1 à 8 pour la préparation d'un médicament destiné au traitement et à la prévention des maladies psychiatriques.

14. Utilisation d'un composé de formule (I) selon l'une quelconque des revendications 1 à 8 pour la préparation d'un médicament destiné au traitement et à la prévention des maladies inflammatoires.

15. Utilisation d'un composé de formule (I) selon l'une quelconque des revendications 1 à 8 pour la préparation d'un médicament destiné au traitement et à la prévention de l'ostéoporose et les cancers.

16. Utilisation d'un composé de formule (I) selon l'une quelconque des revendications 1 à 8 pour la préparation d'un médicament destiné au traitement associé à des greffes et/ou transplantations de cellules souches.

17. Utilisation d'un composé de formule (I) selon l'une quelconque des revendications 1 à 8 pour la préparation d'un médicament destiné au traitement et à la prévention de la maladie de Parkinson, d'Alzheimer, des tauopathies et de la sclérose en plaque.

18. Utilisation d'un composé de formule (I) selon l'une quelconque des revendications 1 à 8 pour la préparation d'un médicament destiné au traitement et à la prévention de la schizophrénie, la dépression, la dépendance à une substance, les troubles du déficit de l'attention et de l'hyperactivité.

19. Procédé d'obtention des composés de formule (I) selon la revendication 1 **caractérisé en ce qu'**ils sont obtenus par une réaction de couplage, catalysée par un métal, entre une 2-arylimidazopyridine de formule générale (II), 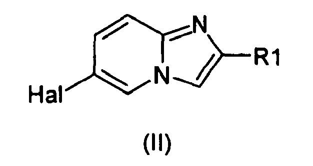 dans laquelle Hal représente un atome d'halogène et R₁ est tel que défini dans la revendication 1,
et un dérivé de formule générale (III), 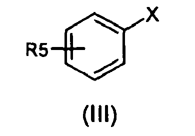 dans laquelle X représente un dérivé de bore ou d'étain et R5 représente le groupe

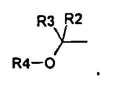

20. Procédé d'obtention des composés de formule (I) selon la revendication 1 **caractérisé en ce qu'**ils sont obtenus par une réaction de couplage catalysée par un métal entre une 2-arylimidazopyridine de formule générale (II), 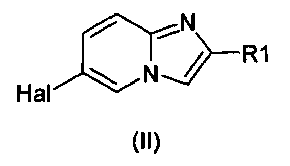 dans laquelle R₁ est tel que défini dans la revendication 1 et Hal représente un atome d'halogène, et un dérivé de formule générale (III), 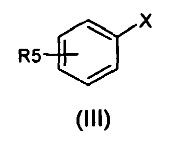 dans laquelle X représente un dérivé de bore ou d'étain et R5 représente un dérivé carbonylé R₂COR₃ , où R2 et R3 sont tels que définis dans la revendication 1, pour obtenir les composés de formule générale (IV), 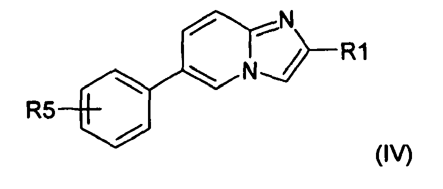 dans laquelle R₁ est tel que défini dans la revendication 1 et R5 représente un dérivé carbonylé R₂COR₃ , où R2 et R3 sont tels que définis dans la revendication 1,
puis en transformant les composés de formule générale (IV) en composés de formule générale (I) par action d'un dérivé organométallique ou par réduction du groupement carbonyle au moyen d'un hydrure métallique.

## Claims

1. Compound corresponding to formula (I): 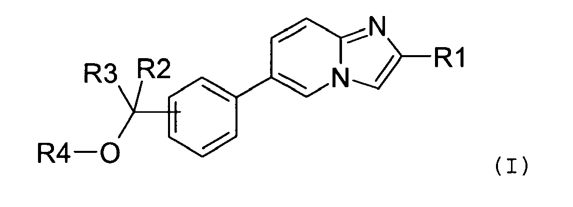 in which:
R₁ is: a phenyl group or a naphthyl group, it being possible for these two groups to be optionally substituted with one or more atoms or groups selected, independently of one another, from the following atoms or groups: halogen, (C₁-C₆) alkyl, (C₃-C₇) cycloalkyl, (C₃-C₇) cycloalkyl (C₁-C₃) alkylene, halo (C₁-C₆) alkyl, (C₁-C₆) alkoxy, (C₃-C₇) cycloalkoxy, (C₃-C₇) cycloalkyl (C₁-C₃) alkyleneoxy, halo (C₁-C₆)-alkoxy, (C₁-C₆) thioalkyl, -S (O) (C₁-C₆) alkyl, -S(O)₂(C₁-C₆-alkyl), hydroxyl, cyano, nitro, hydroxy (C₁-C₆) alkylene, NRaRb(C₁-C₆) alkylene, (C₁-C₆) alkoxy (C₁-C₆) alkyleneoxy, NRaRb, CONRaRb, SO₂NRaRb, NRcCORd, OC(O)NRaRb, NRcC (O) ORe, NRcSO₂Re, aryl (C₁-C₆) alkylene, aryl or heteroaryl, the aryl or the heteroaryl being optionally substituted with one or more substituents selected from a halogen, or a (C₁-C₆) alkyl, (C₃-C₇)cycloalkyl, (C₃-C₇) cycloalkyl (C₁-C₃) alkylene, halo (C₁-C₆) alkyl, (C₁-C₆) alkoxy, halo (C₁-C₆) alkoxy, NRaRb, hydroxyl, nitro or cyano group;
R₂ and R₃ are, independently of one another,
a hydrogen atom,
a (C₁-C₆) alkyl, (C₃-C₇) cycloalkyl or (C₃-C₇) cycloalkyl (C₁-C₃) alkylene group, this group being optionally substituted with an Rf group; an aryl group, the aryl being optionally substituted with one or more substituents selected from a halogen or a (C₁-C₆) alkyl, (C₃-C₇) cycloalkyl, (C₃-C₇) cycloalkyl (C₁-C₃) alkylene, halo(C₁-C₆)alkyl, (C₁-C₆) alkoxy, halo (C₁-C₆) alkoxy, NRaRb, hydroxyl, nitro or cyano group;
R₄ is: a hydrogen atom,
a (C₁-C₆) alkyl, (C₃-C₇) cycloalkyl or (C₃-C₇) cyclo-alkyl (C₁-C₃) alkylene group, this group being optionally substituted with an Rf group;
an aryl group, the aryl being optionally substituted with one or more substituents chosen from a halogen or a (C₁-C₆) alkyl, (C₃-C₇) cycloalkyl, (C₃-C₇) cycloalkyl (C₁-C₃) alkylene, halo (C₁-C₆) alkyl, (C₁-C₆) alkoxy, (C₃-C₇) cycloalkoxy, (C₃-C₇) cyclo-alkyl (C₁-C₃)alkyleneoxy, halo(C₁-C₆)alkoxy, NRaRb, hydroxyl, nitro, cyano, (C₁-C₆) alkyl (CO) -, CONRaRb, NRcCORd, OC(O)NRaRb, NRcC(O)ORe or aryl group, the aryl being optionally substituted with one or more substituents selected from a halogen or a (C₁-C₆) alkyl, (C₃-C₇) cycloalkyl, (C₃-C₇) cyclo-alkyl (C₁-C₃) alkylene, halo(C₁-C₆) alkyl, (C₁-C₆) alkoxy, halo (C₁-C₆) alkoxy, NRaRb, hydroxyl, nitro or cyano group;
Ra and Rb are, independently of one another, a hydrogen atom or a (C₁-C₆)alkyl, (C₃-C₇)cycloalkyl, (C₃-C₇)cycloalkyl(C₁-C₃)alkylene, aryl(C₁-C₆) alkylene or aryl group;
or Ra and Rb form, together with the nitrogen atom which bears them, an azetidine, pyrrolidine, piperidine, azepine, morpholine, thiomorpholine, piperazine or homopiperazine group, this group being optionally substituted with a (C₁-C₆)alkyl, (C₃-C₇) cycloalkyl, (C₃-C₇) cycloalkyl (C₁-C₆) alkylene, aryl or aryl (C₁-C₆) alkylene group;
Rc and Rd are, independently of one another,
a hydrogen atom or a (C₁-C₆) alkyl, (C₃-C₇)cycloalkyl, (C₃-C₇)cycloalkyl(C₁-C₃)alkylene, aryl(C₁-C₆)alkylene and aryl group;
or Rc and Rd together form a (C₂-C₅)alkylene group;
Re is a (C₁-C₆) alkyl, (C₃-C₇) cycloalkyl, (C₃-C₇) cyclo-alkyl (C₁-C₃) alkylene, aryl (C₁-C₆) alkylene and aryl group;
or Rc and Re together form a (C₂-C₅) alkylene group;
Rf is a halogen atom or a (C₁-C₆) alkoxy, halo(C₁-C₆)alkoxy, (C₃-C₇) cycloalkoxy, (C₃-C₇) cycloalkyl (C₁-C₆)-alkyleneoxy, hydroxyl, cyano, NRaRb, C(O)NRaRb, NRcCORd, OC(O)NRaRb, NRcCOORe, SO₂NRaRb, NRcSO₂Re, aryl (C₁-C₆) alkylene or aryl group, the aryl being optionally substituted with one or more substituents selected from a halogen or a (C₁-C₆) alkyl, (C₃-C₇) cycloalkyl, (C₃-C₇) cycloalkyl (C₁-C₃) alkylene, halo (C₁-C₆) alkyl, (C₁-C₆) alkoxy, halo (C₁-C₆) alkoxy, NRaRb, hydroxyl, nitro or cyano group;
in the form of a base or of an addition salt with an acid.

2. Compound of formula (I) according to Claim 1, **characterized in that**:
R₁ is a naphthyl group or a phenyl group which may be optionally substituted with one or more atoms or groups selected, independently of one another, from the following atoms or groups: halogen, (C₁-C₆)alkyl, (C₃-C₇)cycloalkyl, (C₃-C₇)cycloalkyl (C₁-C₆) alkylene, (C₁-C₆) alkoxy, (C₃-C₇)cycloalkyl(C₁-C₆)alkoxy, hydroxyl, cyano, nitro, NRaRb, hydroxy(C₁-C₆)alkyl, (C₁-C₆)alkoxy(C₁-C₆)alkyl, halo(C₁-C₆) alkyl, halo(C₁-C₆) - alkoxy or aryl, the aryl being optionally substituted with one or more substituents selected from a halogen or a (C₁-C₆) alkyl, (C₃-C₇) cycloalkyl, (C₃-C₇) cycloalkyl (C₁-C₃) alkylene, halo (C₁-C₆) alkyl, (C₁-C₆) alkoxy, halo(C₁-C₆)alkoxy, NRaRb, hydroxyl, nitro or cyano group; in the form of a base or of an addition salt with an acid.

3. Compound of formula (I) according to Claim 1 or 2, **characterized in that**:
R₂ and R₃ are, independently of one another,
a hydrogen atom,
a (C₁-C₆) alkyl, (C₃-C₇)cycloalkyl or (C₃-C₇)cyclo-alkyl(C₁-C₃)alkylene group optionally substituted with an Rf group;
R₄ is: a hydrogen atom,
a (C₁-C₆)alkyl, (C₃-C₇)cycloalkyl or (C₃-C₇)cyclo-alkyl(C₁-C₃)alkylene group optionally substituted with an Rf group,
an aryl group optionally substituted with one or more substituents selected from a halogen or a (C₁-C₆) alkyl, (C₃-C₇) cycloalkyl, (C₃-C₇) cyclo-alkyl (C₁-C₃) alkylene, halo(C₁-C₆) alkyl, (C₁-C₆) alkoxy, (C₃-C₇)cycloalkoxy, (C₃-C₇)cyclo-alkyl (C₁-C₃) alkyleneoxy, halo(C₁-C₆)alkoxy, NRaRb, hydroxyl, nitro, cyano, (C₁-C₆)alkyl(CO)-, NRcCORd or aryl group, the aryl being optionally substituted with one or more substituents selected from a halogen or a (C₁-C₆) alkyl, (C₃-C₇)cycloalkyl, (C₃-C₇) cycloalkyl (C₁-C₃) alkylene, halo(C₁-C₆) alkyl, (C₁-C₆) alkoxy, halo (C₁-C₆) alkoxy, NRaRb, hydroxyl, nitro or cyano group;
in the form of a base or of an addition salt with an acid.

4. Compound of formula (I) according to any one of Claims 1 to 3, **characterized in that**:
the substituent 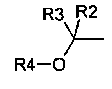 is in the meta-position on the phenyl; in the form of a base or of an addition salt with an acid.

5. Compound of formula (I) according to Claim 1 or 3, **characterized in that**
R₄ is a hydrogen atom and
R₂ and R₃ are, independently of one another, a hydrogen atom or a (C₁-C₆) alkyl, (C₃-C₇)cycloalkyl or (C₃-C₇) cycloalkyl (C₁-C₃) alkylene group optionally substituted with an Rf group;
in the form of a base or of an addition salt with an acid.

6. Compound of formula (I) according to Claim 1, **characterized in that** R₁ is a naphthyl group or a phenyl group which can be optionally substituted with one or more atoms or groups selected, independently of one another, from the following atoms or groups: halogen, (C₁-C₆) alkyl, (C₃-C₇) cycloalkyl, (C₃-C₇) cycloalkyl (C₁-C₆) alkylene, (C₁-C₆) alkoxy, (C₃-C₇) cycloalkyl (C₁-C₆) - alkoxy, hydroxyl, cyano, nitro, NRaRb, hydroxy(C₁-C₆) alkyl, (C₁-C₆) alkoxy (C₁-C₆) alkyl, halo (C₁-C₆) alkyl, halo(C₁-C₆)alkoxy or aryl, the aryl being optionally substituted with one or more substituents selected from a halogen or a (C₁-C₆)alkyl, (C₃-C₇)cycloalkyl, (C₃-C₇)cycloalkyl (C₁-C₃) alkylene, halo(C₁-C₆)alkyl, (C₁-C₆)alkoxy, halo(C₁-C₆)alkoxy, NRaRb, hydroxyl, nitro or cyano group, in the form of a base or of an addition salt with an acid;
R₂ and R₃ are, independently of one another,
a hydrogen atom,
a (C₁-C₆) alkyl, (C₃-C₇)cycloalkyl or (C₃-C₇)cycloalkyl (C₁-C₃)alkylene group, this group being optionally substituted with an Rf group;
an aryl group, the aryl being optionally substituted with one or more substituents selected from a halogen or a (C₁-C₆)alkyl, (C₃-C₇)cycloalkyl, (C₃-C₇) cycloalkyl (C₁-C₃)alkylene, halo(C₁-C₆)alkyl, (C₁-C₆)alkoxy, halo(C₁-C₆)alkoxy, NRaRb, hydroxyl, nitro or cyano group;
R₄ is: a hydrogen atom,
a (C₁-C₆) alkyl, (C₃-C₇)cycloalkyl or (C₃-C₇) cycloalkyl (C₁-C₃)alkylene group, this group being optionally substituted with an Rf group,
an aryl group, the aryl being optionally substituted with one or more substituents selected from a halogen or a (C₁-C₆) alkyl, (C₃-C₇)cycloalkyl, (C₃-C₇) cycloalkyl (C₁-C₃)alkylene, halo(C₁-C₆)alkyl, nitro, cyano, (C₁-C₆)alkyl(CO) - or NRcCORd group;
in the form of a base or of an addition salt with an acid.

7. Compound of formula (I) according to Claim 1,
**characterized in that**
R₁ is a naphthyl group or a phenyl group which can be optionally substituted with one or more atoms or groups selected, independently of one another, from the following atoms or groups: halogen, (C₁-C₆)alkyl, (C₃-C₇) cycloalkyl, (C₃-C₇) cycloalkyl (C₁-C₆) alkylene, (C₁-C₆) alkoxy, (C₃-C₇)cycloalkyl(C₁-C₆)alkoxy, hydroxyl, cyano, nitro, NRaRb, hydroxy(C₁-C₆)alkyl, (C₁-C₆) alkoxy(C₁-C₆) alkyl, halo(C₁-C₆) alkyl or halo(C₁-C₆) alkoxy,
R₂ and R₃ are, independently of one another,
a hydrogen atom,
a (C₁-C₆) alkyl, (C₃-C₇) cycloalkyl or (C₃-C₇) cycloalkyl (C₁-C₃) alkylene group, this group being optionally substituted with an Rf group;
an aryl group, the aryl being optionally substituted with one or more substituents selected from a halogen or a (C₁-C₆) alkyl, (C₃-C₇)cycloalkyl, (C₃-C₇) cycloalkyl (C₁-C₃) alkylene, halo (C₁-C₆) alkyl, (C₁-C₆) alkoxy, halo(C₁-C₆) alkoxy, NRaRb, hydroxyl, nitro or cyano group;
R₄ is:
a hydrogen atom,
a (C₁-C₆) alkyl, (C₃-C₇) cycloalkyl or (C₃-C₇) cycloalkyl (C₁-C₃) alkylene group, this group being optionally substituted with an Rf group, an aryl group, the aryl being optionally substituted with one or more substituents selected from a halogen or a (C₁-C₆) alkyl, (C₃-C₇)cycloalkyl, (C₃-C₇) cycloalkyl (C₁-C₃) alkylene, halo (C₁-C₆) alkyl, nitro, cyano, (C₁-C₆) alkyl (CO) - or NRcCORd group;
in the form of a base or of an addition salt with an acid.

8. Compounds of formula (I) according to Claim 1, chosen from the following compounds:
[4-(2-phenylimidazo[1,2*-a*]pyridin-6-yl)phenyl]methanol
[3-(2-phenylimidazo[1,2*-a*]pyridin-6-yl)phenyl]methanol
{4-[2-(4-pyrrolidin-1-ylphenyl)imidazo[1,2*-a*] pyridin-6-yl]phenyl}methanol
[4-(2-biphenyl-4-ylimidazo[1,2*-a*]pyridin-6-yl)phenyl]methanol
3-[6-(4-hydroxymethylphenyl)imidazo[1,2*-a*]pyridin-2-yl]benzonitrile
3-[6-(3-hydroxymethylphenyl)imidazo[1,2*-a*]pyridin-2-yl]benzonitrile
3-{6-[3-(2-methoxyethoxymethyl)phenyl]imidazo[1,2-a]pyridin-2-yl}benzonitrile
4-[6-(3-hydroxymethylphenyl)imidazo[1,2*-a*]pyridin-2-yl]benzonitrile
[3-[2-(naphthalen-2-yl)imidazo[1,2*-a*]pyridin-6-yl]phenyl]methanol
[3-(2*-p*-tolylimidazo[1,2*-a*]pyridin-6-yl)phenyl]methanol
{3-[2-(4-pyrrolidin-1-ylphenyl)imidazo[1,2*-a*]pyridin-6-yl]phenyl}methanol
{3-[2-(3-trifluoromethoxyphenyl)imidazo[1,2*-a*] pyridin-6-yl]phenyl}methanol
{3-[2-(4-nitrophenyl)imidazo[1,2*-a*]pyridin-6-yl]phenyl}methanol
{4-[2-(4-diethylaminophenyl)imidazo[1,2*-a*]pyridin-6-yl]phenyl}methanol
[3-(2-naphthalen-1-ylimidazo[1,2*-a*]pyridin-6-yl)phenyl]methanol
{3-[2-(2,4-dimethylphenyl)imidazo[1,2*-a*]pyridin-6-yl]phenyl}methanol
6-[3-(2-methoxyethoxymethyl)phenyl]-2-naphthalen-2-ylimidazo[1,2*-a*]pyridine
{3-[2-(4-methoxyphenyl)imidazo[1,2*-a*]pyridin-6-yl]phenyl}methanol
{3-[2-(3-methoxyphenyl)imidazo[1,2*-a*]pyridin-6-yl]phenyl}methanol
{3-[2-(2-methoxyphenyl)imidazo[1,2*-a*]pyridin-6-yl]phenyl}methanol
{3-[2-(2,4-dimethoxyphenyl)imidazo[1,2*-a*]pyridin-6-yl]phenyl}methanol
{3-[2-(4-fluorophenyl)imidazo[1,2*-a*]pyridin-6-yl]phenyl}methanol
{3-[2-(3,5-difluorophenyl)imidazo[1,2*-a*]pyridin-6-yl]phenyl}methanol
{3-[2-(3-fluoro-5-trifluoromethylphenyl)imidazo[1,2-a]pyridin-6-yl]phenyl}methanol
{3-[2-(4-chlorophenyl)imidazo[1,2*-a*]pyridin-6-yl]phenyl}methanol
{3-[2-(4-chlorophenyl)imidazo[1,2*-a*]pyridin-6-yl]phenyl}methanol hydrochloride (1:1)
{3-[2-(3-chlorophenyl)imidazo[1,2*-a*]pyridin-6-yl]phenyl}methanol
{3-[2-(4-chloro-3-methylphenyl)imidazo[1,2*-a*]pyridin-6-yl]phenyl}methanol
{3-[2-(3,4-dichlorophenyl)imidazo[1,2*-a*]pyridin-6-yl]phenyl}methanol
{3-[2-(3-fluorophenyl)imidazo[1,2*-a*]pyridin-6-yl]-phenyl}methanol
6-[3-(4-chlorophenoxymethyl)phenyl]-2*-p*-tolylimidazo[1,2*-a*]pyridine
*N*-{4-[3-(2*-p*-tolylimidazo[1,2*-a*]pyridin-6-yl)-benzyloxy]phenyl}acetamide
6-[3-(3-nitrophenoxymethyl)phenyl]-2*-p-*tolylimidazo[1,2*-a*]pyridine
6-[3-(4-methylphenoxymethyl)phenyl]-2*-p*-tolylimidazo[1,2*-a*]pyridine
4-[3-(2*-p*-tolylimidazo[1,2*-a*]pyridin-6-yl)benzyloxy]-benzonitrile
1-{4-[3-(2*-p*-tolylimidazo[1,2*-a*]pyridin-6-yl)benzyloxy]phenyl}ethanone
6-[3-(4-trifluoromethylphenoxymethyl)phenyl]-2*-p-*tolylimidazo[1,2*-a*]pyridine
{3-[2-(3-fluoro-4-methylphenyl)imidazo[1,2*-a*]pyridin-6-yl]phenyl}methanol
2-{3-[2-(4-chlorophenyl)imidazo[1,2*-a*]pyridin-6-yl]phenyl}propan-2-ol
{2-[2-(4-chlorophenyl)imidazo[1,2*-a*]pyridin-6-yl]-phenyl}methanol
{2-[2-(4-chlorophenyl)imidazo[1,2*-a*]pyridin-6-yl]phenyl}methanol hydrochloride (1:1)
racemic 1-{3-[2-(4-chlorophenyl)imidazo[1,2*-a*]pyridin-6-yl]phenyl}ethanol
{3-[2-(2,4-dichlorophenyl)imidazo[1,2*-a*]pyridin-6-yl]-phenyl}methanol
{3-[2-(2,4-difluorophenyl)imidazo[1,2*-a*]pyridin-6-yl]-phenyl}methanol
{3-[2-(3,4-difluorophenyl)imidazo[1,2*-a*]pyridin-6-yl]-phenyl}methanol
{3-[2-(3,4-difluorophenyl)imidazo[1,2*-a*]pyridin-6-yl]-phenyl}methanol hydrochloride (1:1)
{3-[2-(2-chlorophenyl)imidazo[1,2*-a*]pyridin-6-yl]-phenyl}methanol
{3-[2-(4-trifluoromethylphenyl)imidazo[1,2*-a*]pyridin-6-yl]phenyl}methanol
{3-[2-(4-(difluoromethyl)phenyl)imidazo[1,2*-a*]pyridin-6-yl]phenyl}methanol
racemic 1-{2-[2-(4-chlorophenyl)imidazo[1,2*-a*]pyridin-6-yl]phenyl}ethanol
2-(4-chlorophenyl)-6-(2-methoxymethyl-phenyl)imidazo[1,2*-a*]pyridine
2-(4-chlorophenyl)-6-(2-methoxymethyl-phenyl)imidazo[1,2*-a*]pyridine hydrochloride (1:1)
2-(4-chlorophenyl)-6-(4-methoxymethylphenyl)imidazo[1,2*-a*]pyridine
racemic 1-{3-[2-(4-chlorophenyl)imidazo[1,2*-a*]pyridin-6-yl]phenyl}propan-1-ol
racemic 1-{3-[2-(4-chlorophenyl)imidazo[1,2*-a*]pyridin-6-yl]phenyl}pentan-1-ol
racemic 1-{3-[2-(4-chlorophenyl)imidazo[1,2*-a*] pyridin-6-yl]phenyl}heptan-1-ol
racemic 1-{3-[2-(4-chlorophenyl)imidazo[1,2*-a*]pyridin-6-yl]phenyl}-3-methylbutan-1-ol
racemic {3-[2-(4-chlorophenyl)imidazo[1,2*-a*]pyridin-6-yl]phenyl}cyclopentylmethanol
racemic {3-[2-(4-chlorophenyl)imidazo[1,2*-a*]pyridin-6-yl]phenyl}phenylmethanol
dextrorotatory enantiomer of 1-{3-[2-(4-chlorophenyl)imidazo[1,2*-a*]pyridin-6-yl]phenyl}ethanol
levorotatory enantiomer of 1-{3-[2-(4-chlorophenyl)imidazo[1,2*-a*]pyridin-6-yl]phenyl}ethanol
2-(4-chlorophenyl)-6-(3-methoxymethylphenyl)imidazo[1,2*-a*]pyridine
racemic 1-{4-[2-(4-chlorophenyl)imidazo[1,2*-a*]pyridin-6-yl]phenyl}ethanol
racemic 1-{3-[2-(2,4-difluorophenyl)imidazo[1,2-a]pyridin-6-yl]phenyl}ethanol
racemic 1-{3-[2-(4-fluorophenyl)imidazo[1,2*-a*]pyridin-6-yl]phenyl}ethanol
racemic 1-{3-[2-(3,4-difluorophenyl)imidazo[1,2-a]pyridin-6-yl]phenyl}ethanol
racemic 1-{3-[2-(2-chlorophenyl)imidazo[1,2*-a*]pyridin-6-yl]phenyl}ethanol
racemic 1-{3-[2-(3-fluorophenyl)imidazo[1,2*-a*]pyridin-6-yl]phenyl}ethanol
racemic 1-{3-[2-(3-chlorophenyl)imidazo[1,2*-a*]pyridin-6-yl]phenyl}ethanol
4-[6-(3-hydroxymethylphenyl)imidazo[1,2*-a*]pyridin-2-yl]phenol
in the form of a base or of an addition salt.

9. Medicament, **characterized in that** it comprises a compound of formula (I) according to any one of Claims 1 to 8, or an addition salt of this compound with a pharmaceutically acceptable acid.

10. Pharmaceutical composition, **characterized in that** it comprises a compound of formula (I) according to any one of Claims 1 to 8, or a pharmaceutically acceptable salt of this compound, and also at least one pharmaceutically acceptable excipient.

11. Use of a compound of formula (I) according to any one of Claims 1 to 8, for the preparation of a medicament for use in the treatment or prevention of neurodegenerative diseases.

12. Use of a compound of formula (I) according to any one of Claims 1 to 8, for the preparation of a medicament for use in the treatment or prevention of cerebral traumas and epilepsy.

13. Use of a compound of formula (I) according to any one of Claims 1 to 8, for the preparation of a medicament for use in the treatment or prevention of psychiatric diseases.

14. Use of a compound of formula (I) according to any one of Claims 1 to 8, for the preparation of a medicament for use in the treatment or prevention of inflammatory diseases.

15. Use of a compound of formula (I) according to any one of Claims 1 to 8, for the preparation of a medicament for use in the treatment or prevention of osteoporosis and cancers.

16. Use of a compound of formula (I) according to any one of Claims 1 to 8, for the preparation of a medicament for use in treatment combined with stem cell transplants and/or grafts.

17. Use of a compound of formula (I) according to any one of Claims 1 to 8, for the preparation of a medicament for use in the treatment or prevention of Parkinson's Disease, Alzheimer's Disease, tauopathies and multiple sclerosis.

18. Use of a compound of formula (I) according to any one of Claims 1 to 8, for the preparation of a medicament for use in the treatment or prevention of schizophrenia, depression, substance dependency and attention deficit hyperactivity disorders.

19. Process for obtaining the compounds of formula (I) according to Claim 1, **characterized in that** they are obtained by means of a coupling reaction, catalysed by a metal, between a 2-arylimidazopyridine of general formula (II): 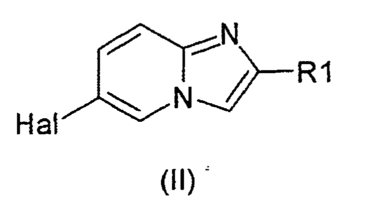 in which Hal is a halogen atom and R₁ is as defined in Claim 1,
and a derivative of general formula (III): 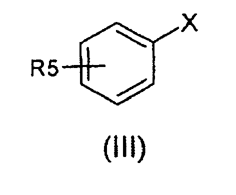 in which X is a derivative of boron or of tin, and R₅ is the 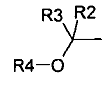 group.

20. Process for obtaining the compounds of formula (I) according to Claim 1, **characterized in that** they are obtained by means of a coupling reaction, catalysed by a metal, between a 2-arylimidazopyridine of general formula (II): 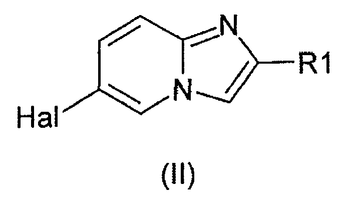 in which R₁ is as defined in Claim 1 and Hal is a halogen atom,
and a derivative of general formula (III): 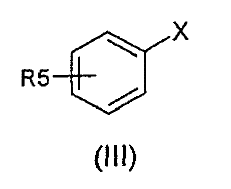 in which X is a derivative of boron or of tin, and R₅ is a carbonyl derivative R₂COR₃, where R₂ and R₃ are as defined in Claim 1,
so as to obtain the compounds of general formula (IV): 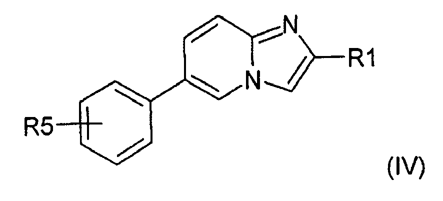 in which R₁ is as defined in Claim 1 and R₅ is a carbonyl derivative R₂COR₃, where R₂ and R₃ are as defined in Claim 1,
R₂ and R₃ are as defined in Claim 1, and then by converting the compounds of general formula (IV) to compounds of general formula (I) through the action of an organometallic derivative or by reduction of the carbonyl group by means of a metal hydride.

## Patentansprüche

1. Verbindung der Formel (I) : 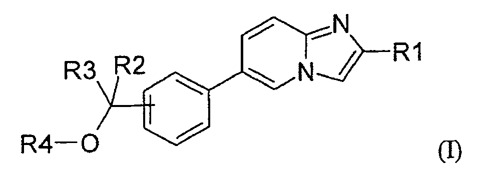 worin
R₁ für
eine Phenylgruppe oder eine Naphthylgruppe steht, wobei diese beiden Gruppen gegebenenfalls durch ein oder mehrere Atome bzw. eine oder mehrere Gruppen substituiert sein können, die unabhängig voneinander unter den folgenden Atomen bzw. Gruppen ausgewählt sind: Halogen, (C₁-C₆) -Alkyl, (C₃-C₇) -Cycloalkyl, (C₃-C₇)-Cycloalkyl-(C₁-C₃)-alkylen, Halogen-(C₁-C₆)-alkyl, (C₁-C₆)-Alkoxy, (C₃-C₇) -Cycloalkoxy, (C₃-C₇)-Cycloalkyl-(C₁-C₃)-alkylenoxy, Halogen-(C₁-C₆)-alkoxy, (C₁-C₆) -Thioalkyl, -S (O) - (C₁-C₆)-Alkyl, -S(O)₂-(C₁-C₆-Alkyl), Hydroxyl, Cyano, Nitro, Hydroxy-(C₁-C₆)-alkylen, NRaRb- (C₁-C₆)-Alkylen, (C₁-C₆)-Alkoxy- (C₁-C₆) -alkylenoxy, NRaRb, CONRaRb, SO₂NRaRb, NRcCORd, OC(O)NRaRb, NRcC(O)ORe, NRcSO₂Re, Aryl-(C₁-C₆)-alkylen, Aryl oder Heteroaryl, wobei das Aryl oder Heteroaryl gegebenenfalls durch einen oder mehrere unter einem Halogen oder einer (C₁-C₆) -Alkyl-, (C₃-C₇)-Cycloalkyl-, (C₃-C₇) -Cycloalkyl- (C₁-C₃) -alkylen-, Halogen-(C₁-C₆)-alkyl-, (C₁-C₆)-Alkoxy-, Halogen-(C₁-C₆)-alkoxy-, NRaRb-, Hydroxyl-, Nitro- oder Cyanogruppe ausgewählte Substituenten substituiert ist;
R₂ und R₃ unabhängig voneinander für
ein Wasserstoffatom,
eine (C₁-C₆) -Alkyl-, (C₃-C₇) -Cycloalkyl- oder (C₃-C₇)-Cycloalkyl-(C₁-C₃)-alkylengruppe, die gegebenenfalls durch eine Gruppe Rf substituiert ist;
eine Arylgruppe, wobei das Aryl gegebenenfalls durch einen oder mehrere unter einem Halogen oder einer (C₁-C₆) -Alkyl-, (C₃-C₇) -Cycloalkyl-, (C₃-C₇)-Cycloalkyl-(C₁-C₃)-alkylen-, Halogen-(C₁-C₆) -alkyl-, (C₁-C₆) -Alkoxy-, Halogen- (C₁-C₆)-alkoxy-, NRaRb-, Hydroxyl-, Nitro- oder Cyanogruppe ausgewählte Substituenten substituiert ist,
stehen;
R₄ für
ein Wasserstoffatom,
eine (C₁-C₆) -Alkyl-, (C₃-C₇)-Cycloalkyl- oder (C₃-C₇)-Cycloalkyl-(C₁-C₃)-alkylengruppe, die gegebenenfalls durch eine Gruppe Rf substituiert ist;
eine Arylgruppe, wobei das Aryl gegebenenfalls durch einen oder mehrere unter einem Halogen oder einer (C₁-C₆) -Alkyl-, (C₃-C₇) -Cycloalkyl-, (C₃-C₇) -Cycloalkyl- (C₁-C₃) -alkylen-, Halogen- (C₁-C₆) -alkyl-, (C₁-C₆) -Alkoxy-, (C₃-C₇)-Cycloalkyloxy-, (C₃-C₇)-Cycloalkyl-(C₁-C₃)-alkylenoxy-, Halogen-(C₁-C₆)-alkoxy-, NRaRb-, Hydroxyl-, Nitro-, Cyano-, (C₁-C₆) -Alkyl- (CO) -, CONRaRb-, NRcCORd-, OC(O)NRaRb-, NRcC(O)ORe-oder Arylgruppe ausgewählte Substituenten substituiert ist, wobei das Aryl gegebenenfalls durch einen oder mehrere unter einem Halogen oder einer (C₁-C₆) -Alkyl-, (C₃-C₇)-Cycloalkyl-, (C₃-C₇) -Cycloalkyl- (C₁-C₃) -alkylen-, Halogen- (C₁-C₆) -alkyl-, (C₁-C₆) -Alkoxy-, Halogen- (C₁-C₆)-alkoxy-, NRaRb-, Hydroxyl-, Nitro- oder Cyanogruppe ausgewählte Substituenten substituiert ist,
steht;
Ra und Rb unabhängig voneinander für
ein Wasserstoffatom oder eine (C₁-C₆)-Alkyl-, (C₃-C₇) -Cycloalkyl-, (C₃-C₇) -Cycloalkyl- (C₁-C₃)-alkylen-, Aryl-(C₁-C₆)-alkylen- oder Arylgruppe
stehen;
oder Ra und Rb gemeinsam mit dem Stickstoffatom, an das sie gebunden sind, eine Azetidin-, Pyrrolidin-, Piperidin-, Azepin-, Morpholin-, Thiomorpholin-, Piperazin- oder Homopiperazingruppe bilden, die gegebenenfalls durch eine (C₁-C₆)-Alkyl-, (C₃-C₇) -Cycloalkyl-, (C₃-C₇)-Cycloalkyl-(C₁-C₆)-alkylen-, Aryl- oder Aryl-(C₁-C₆)-alkylengruppe substituiert ist;
Rc und Rd unabhängig voneinander für
ein Wasserstoffatom oder eine (C₁-C₆)-Alkyl-, (C₃-C₇) -Cycloalkyl-, (C₃-C₇) -Cycloalkyl- (C₁-C₃)-alkylen-, Aryl-(C₁-C₆)-alkylen- oder Arylgruppe
stehen;
oder Rc und Rd gemeinsam eine (C₂-C₅)-Alkylengruppe bilden;
Re für
eine (C₁-C₆)-Alkyl-, (C₃-C₇)-Cycloalkyl-, (C₃-C₇) -Cycloalkyl- (C₁-C₃) -alkylen-, Aryl-(C₁-C₆)-alkylen- oder Arylgruppe
steht;
oder Rc und Re gemeinsam eine (C₂-C₅)-Alkylengruppe bilden;
Rf für
ein Wasserstoffatom oder eine (C₁-C₆)-Alkoxy-, Halogen-(C₁-C₆)-alkoxy-, (C₃-C₇) -Cycloalkoxy-, (C₃-C₇) -Cycloalkyl- (C₁-C₆) -alkylenoxy-, Hydroxyl-, Cyano-, NRaRb-, C(O)NRaRb-, NRcCORd-, OC(O)NRaRb-, NRcCOORe-, SO₂NRaRb-NRcSO₂Re-, Aryl-(C₁-C₆) -alkylen- oder Arylgruppe, wobei das Aryl gegebenenfalls durch einen oder mehrere unter einem Halogen oder einer (C₁-C₆) -Alkyl-, (C₃-C₇) -Cycloalkyl-, (C₃-C₇) -Cycloalkyl- (C₁-C₃) -alkylen-, Halogen- (C₁-C₆) -alkyl-, (C₁-C₆) -Alkoxy-, Halogen- (C₁-C₆)-alkoxy-, NRaRb-, Hydroxyl-, Nitro- oder Cyano-gruppe ausgewählte Substituenten substituiert ist,
steht;
in Basen- oder Säureadditionssalzform.

2. Verbindung der Formel (I) nach Anspruch 1, **dadurch gekennzeichnet, daß**
R₁ für eine Naphthylgruppe oder eine Phenylgruppe steht, die gegebenenfalls durch ein oder mehrere Atome bzw. eine oder mehrere Gruppen substituiert sein kann, die unabhängig voneinander unter den folgenden Atomen bzw. Gruppen ausgewählt sind: Halogen, (C₁-C₆) -Alkyl, (C₃-C₇)-Cycloalkyl, (C₃-C₇-Cycloalkyl- (C₁-C₆)-alkylen, (C₁-C₆)-Alkoxy, (C₃-C₇-Cycloalkyl-(C₁-C₆)-alkoxy, Hydroxyl, Cyano, Nitro, NRaRb, Hydroxy-(C₁-C₆)-alkyl, (C₁-C₆) -Alkoxy- (C₁-C₆) -alkyl, Halogen- (C₁-C₆) -alkyl, Halogen- (C₁-C₆) - alkoxy oder Aryl, wobei das Aryl gegebenenfalls durch einen oder mehrere unter einem Halogen oder einer (C₁-C₆) -Alkyl-, (C₃-C₇)-Cycloalkyl-, (C₃-C₇) - Cycloalkyl-(C₁-C₃)-alkylen-, Halogen-(C₁-C₆)-alkyl-, (C₁-C₆)-Alkoxy-, Halogen-(C₁-C₆) -alkoxy-, NRaRb-, Hydroxyl-, Nitro- oder Cyanogruppe ausgewählte Substituenten substituiert ist; in Basen- oder Säureadditionssalzform.

3. Verbindung der Formel (I) nach Anspruch 1 oder 2,
**dadurch gekennzeichnet, daß**
R₂ und R₃ unabhängig voneinander für
ein Wasserstoffatom,
eine (C₁-C₆) -Alkyl-, (C₃-C₇) -Cycloalkyl- oder (C₃-C₇)-Cycloalkyl-(C₁-C₃)-alkylengruppe, die gegebenenfalls durch eine Gruppe Rf substituiert ist,
stehen;
R₄ für
ein Wasserstoffatom,
eine (C₁-C₆) -Alkyl-, (C₃-C₇) -Cycloalkyl- oder (C₃-C₇)-Cycloalkyl-(C₁-C₃)-alkylengruppe, die gegebenenfalls durch eine Gruppe Rf substituiert ist;
eine Arylgruppe, die gegebenenfalls durch einen oder mehrere unter einem Halogen oder einer (C₁-C₆) -Alkyl-, (C₃-C₇) -Cycloalkyl-, (C₃-C₇)-Cycloalkyl- (C₁-C₃) -alkylen-, Halogen- (C₁-C₆)-alkyl-, (C₁-C₆)-Alkoxy-, (C₃-C₇) -Cycloalkoxy-, (C₃-C₇)-Cycloalkyl-(C₁-C₃)-alkylenoxy-, Halogen-(C₁-C₆)-alkoxy-, NRaRb-, Hydroxyl-, Nitro-, Cyano-, (C₁-C₆)-Alkyl-(CO)-, NRcCORd- oder Arylgruppe ausgewählte Substituenten substituiert ist, wobei das Aryl gegebenenfalls durch einen oder mehrere unter einem Halogen oder einer (C₁-C₆)-Alkyl-, (C₃-C₇)-Cycloalkyl-, (C₃-C₇)-Cycloalkyl- (C₁-C₃)-alkylen-, Halogen- (C₁-C₆)-alkyl-, (C₁-C₆) -Alkoxy-, Halogen- (C₁-C₆)-alkoxy-, NRaRb-, Hydroxyl-, Nitro- oder Cyanogruppe ausgewählte Substituenten substituiert ist,
steht;
in Basen- oder Säureadditionssalzform.

4. Verbindung der Formel (I) nach einem der Ansprüche 1 bis 3, **dadurch gekennzeichnet, daß** der Substituent 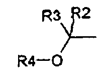 in meta-Stellung am Phenyl steht; in Basen- oder Säureadditionssalzform.

5. Verbindung der Formel (I) nach Anspruch 1 oder 3, **dadurch gekennzeichnet, daß**
R₄ für ein Wasserstoffatom steht und
R₂ und R₃ unabhängig voneinander für ein Wasserstoffatom oder eine (C₁-C₆)-Alkyl-, (C₃-C₇)-Cycloalkyl- oder (C₃-C₇)-Cycloalkyl-(C₁-C₃)-alkylengruppe, die gegebenenfalls durch eine Gruppe Rf substituiert ist, stehen;
in Basen- oder Säureadditionssalzform.

6. Verbindung der Formel (I) nach Anspruch 1, **dadurch gekennzeichnet, daß** R₁ für eine Naphthylgruppe oder eine Phenylgruppe steht, die gegebenenfalls durch ein oder mehrere Atome bzw. eine oder mehrere Gruppen substituiert sein kann, die unabhängig voneinander unter den folgenden Atomen bzw. Gruppen ausgewählt sind: Halogen, (C₁-C₆)-Alkyl, (C₃-C₇) -Cycloalkyl, (C₃-C₇)-Cycloalkyl-(C₁-C₆) - alkylen, (C₁-C₆)-Alkoxy, (C₃-C₇)-Cycloalkyl-(C₁-C₆) - alkoxy, Hydroxyl, Cyano, Nitro, NRaRb, Hydroxy-(C₁-C₆) -alkyl, (C₁-C₆) -Alkoxy- (C₁-C₆) -alkyl, Halogen-(C₁-C₆)-alkyl, Halogen-(C₁-C₆) -alkoxy oder Aryl,
wobei das Aryl gegebenenfalls durch einen oder mehrere unter einem Halogen oder einer (C₁-C₆)-Alkyl-, (C₃-C₇)-Cycloalkyl-, (C₃-C₇)-Cycloalkyl-(C₁-C₃) -alkylen-, Halogen- (C₁-C₆) -alkyl-, (C₁-C₆)-Alkoxy-, Halogen-(C₁-C₆)-alkoxy-, NRaRb-, Hydroxyl-, Nitro- oder Cyanogruppe ausgewählte Substituenten substituiert ist, in Basen- oder Säureadditionssalzform;
R₂ und R₃ unabhängig voneinander für ein Wasserstoffatom,
eine (C₁-C₆) -Alkyl-, (C₃-C₇) -Cycloalkyl- oder (C₃-C₇)-Cycloalkyl-(C₁-C₃) -alkylengruppe, die gegebenenfalls durch eine Gruppe Rf substituiert ist,
eine Arylgruppe, wobei das Aryl gegebenenfalls durch einen oder mehrere unter einem Halogen oder einer (C₁-C₆)-Alkyl-, (C₃-C₇) -Cycloalkyl-, (C₃-C₇) -Cycloalkyl- (C₁-C₃) -alkylen-, Halogen- (C₁-C₆) -alkyl-, (C₁-C₆) -Alkoxy-, Halogen- (C₁-C₆) - alkoxy-, NRaRb-, Hydroxyl-, Nitro- oder Cyanogruppe ausgewählte Substituenten sub-stituiert ist,
stehen;
R₄ für
ein Wasserstoffatom,
eine (C₁-C₆)-Alkyl-, (C₃-C₇)-Cycloalkyl- oder (C₃-C₇)-Cycloalkyl-(C₁-C₃) -alkylengruppe, die gegebenenfalls durch eine Gruppe Rf substituiert ist;
eine Arylgruppe, die gegebenenfalls durch einen oder mehrere unter einem Halogen oder einer (C1-C₆)-Alkyl-, (C₃-C₇)-Cycloalkyl-, (C₃-C₇)-Cycloalkyl-(C₁-C₃)-alkylen-, Halogen-(C₁-C₆)-alkyl-, Nitro-, Cyano-, (C₁-C₆) -Alkyl- (CO) - oder NRcCORd-Gruppe ausgewählte Substituenten substituiert ist,
steht;
in Basen- oder Säureadditionssalzform.

7. Verbindung der Formel (I) nach Anspruch 1, **dadurch gekennzeichnet, daß**
R₁ für eine Naphthylgruppe oder eine Phenylgruppe steht, die gegebenenfalls durch ein oder mehrere Atome bzw. eine oder mehrere Gruppen substituiert sein kann, die unabhängig voneinander unter den folgenden Atomen bzw. Gruppen ausgewählt sind: Halogen, (C₁-C₆)-Alkyl, (C₃-C₇-Cycloalkyl, (C₃-C₇) - Cycloalkyl-(C₁-C₆)-alkylen, (C₁-C₆)-Alkoxy, (C₃-C₇)-Cycloalkyl-(C₁-C₆)-alkoxy, Hydroxyl, Cyano, Nitro, NRaRb, Hydroxy- (C₁-C₆) -alkyl, (C₁-C₆) -Alkoxy- (C₁-C₆)-alkyl, Halogen- (C₁-C₆) -alkyl, oder Halogen- (C₁-C₆)-alkoxy,
R₂ und R₃ unabhängig voneinander für
ein Wasserstoffatom,
eine (C₁-C₆)-Alkyl-, (C₃-C₇)-Cycloalkyl- oder (C₃-C₇)-Cycloalkyl-(C₁-C₃)-alkylengruppe, die gegebenenfalls durch eine Gruppe Rf substituiert ist,
eine Arylgruppe, wobei das Aryl gegebenenfalls durch einen oder mehrere unter einem Halogen oder einer (C₁-C₆)-Alkyl-, (C₃-C₇)-Cycloalkyl-, (C₃-C₇)-Cycloalkyl-(C₁-C₃)-alkylen-, Halogen-(C₁-C₆)-alkyl-, (C₁-C₆)-Alkoxy-, Halogen-(C₁-C₆)-alkoxy-, NRaRb-, Hydroxyl-, Nitro- oder Cyanogruppe ausgewählte Substituenten substituiert ist,
stehen;
R₄ für
ein Wasserstoffatom,
eine (C₁-C₆) -Alkyl-, (C₃-C₇)-Cycloalkyl- oder (C₃-C₇)-Cycloalkyl-(C₁-C₃)-alkylengruppe, die gegebenenfalls durch eine Gruppe Rf substituiert ist;
eine Arylgruppe, die gegebenenfalls durch einen oder mehrere unter einem Halogen oder einer (C₁-C₆)-Alkyl-, (C₃-C₇)-Cycloalkyl-, (C₃-C₇)-Cycloalkyl- (C₁-C₃) -alkylen-, Halogen- (C₁-C₆)-alkyl-, Nitro-, Cyano-, (C₁-C₆)-Alkyl- (CO) - oder NRcCORd-Gruppe ausgewählte Substituenten substituiert ist,
steht;
in Basen- oder Säureadditionssalzform.

8. Verbindungen der Formel (I) nach Anspruch 1, ausgewählt unter den folgenden Verbindungen:
[4-(2-Phenylimidazo[1,2-*a*]pyridin-6-yl)phenyl]-methanol
[3-(2-Phenylimidazo[1,2-*a*]pyridin-6-yl)phenyl]-methanol
{4-[2-(4-Pyrrolidin-1-ylphenyl)imidazo[1,2-*a*]-pyridin-6-yl]phenyl}methanol
[4-(2-Biphenyl-4-ylimidazo[1,2-*a*]pyridin-6-yl)-phenyl]methanol
3-[6-(4-Hydroxymethylphenyl)imidazo[1,2-*a*]pyridin-2-yl]benzonitril
3-[6-(3-Hydroxymethylphenyl)imidazo[1,2-*a*]pyridin-2-yl]benzonitril
3-{6-[3-(2-Methoxyethoxymethyl)phenyl]imidazo[1,2-*a*]pyridin-2-yl}benzonitril
4-[6-(3-Hydroxymethylphenyl)imidazo[1,2-*a*]pyridin-2-yl]benzonitril
[3-[2-(Naphthalin-2-yl)imidazo[1,2-*a*]pyridin-6-yl]phenyl]methanol
[3-(2-*p*-Tolylimidazo[1,2-*a*]pyridin-6-yl)phenyl]-methanol
{3-[2-(4-Pyrrolidin-1-ylphenyl)imidazo[1,2-*a*]-pyridin-6-yl]phenyl}methanol
{3-[2-(3-Trifluormethoxyphenyl)imidazo[1,2-*a*]-pyridin-6-yl]phenyl}methanol
{3-[2-(4-Nitrophenyl)imidazo[1,2-*a*]pyridin-6-yl]-phenyl}methanol
{4-[2-(4-Diethylaminophenyl)imidazo[1,2-*a*]pyridin-6-yl]phenyl}methanol
[3-(2-Naphthalin-1-ylimidazo[1,2-*a*]pyridin-6-yl)-phenyl]methanol
{3-[2-(2,4-Dimethylphenyl)imidazo[1,2-*a*]pyridin-6-yl]phenyl}methanol
6-[3-(2-Methoxyethoxymethyl)phenyl]-2-naphthalin-2-ylimidazo[1,2-*a*]pyridin
{3-[2-(4-Methoxyphenyl)imidazo[1,2-*a*]pyridin-6-yl]phenyl}methanol
{3-[2-(3-Methoxyphenyl)imidazo[1,2-*a*]pyridin-6-yl]phenyl}methanol
{3-[2-(2-Methoxyphenyl)imidazo[1,2-*a*]pyridin-6-yl]phenyl}methanol
{3-[2-(2,4-Dimethoxyphenyl)imidazo[1,2-*a*]pyridin-6-yl]phenyl}methanol
{3-[2-(4-Fluorphenyl)imidazo[1,2-*a*]pyridin-6-yl]-phenyl}methanol
{3-[2-(3,5-Difluorphenyl)imidazo[1,2-*a*]pyridin-6-yl]phenyl}methanol
{3-[2-(3-Fluor-5-trifluormethylphenyl)imidazo[1,2-a]pyridin-6-yl]phenyl}methanol
{3-[2-(4-Chlorphenyl)imidazo[1,2-*a*]pyridin-6-yl]-phenyl}methanol
{3-[2-(4-Chlorphenyl)imidazo[1,2-*a*]pyridin-6-yl]-phenyl}methanol-hydrochlorid (1:1)
{3-[2-(3-Chlorphenyl)imidazo[1,2-*a*]pyridin-6-yl]-phenyl}methanol
{3-[2-(4-Chlor-3-methylphenyl)imidazo[1,2-*a*]-pyridin-6-yl]phenyl}methanol
{3-[2-(3,4-Dichlorphenyl)imidazo[1,2-*a*]pyridin-6-yl]phenyl}methanol
{3-[2-(3-Fluorphenyl)imidazo[1,2-*a*]pyridin-6-yl]-phenyl}methanol
6-[3-(4-Chlorphenoxymethyl)phenyl]-2-*p*-tolyl-imidazo[1,2-*a*]pyridin
*N*-{4-[3-(2-*p*-Tolylimidazo[1,2-*a*]pyridin-6-yl)-benzyloxy]phenyl}acetamid
6-[3-(3-Nitrophenoxymethyl)phenyl]-2-*p*-tolyl-imidazo[1,2-*a*]pyridin
6-[3-(4-Methylphenoxymethyl)phenyl]-2-*p*-tolyl-imidazo[1,2-*a*]pyridin
4-[3-(2-*p*-Tolylimidazo[1,2-*a*]pyridin-6-yl)benzyl-oxy]benzonitril
1-{4- [3-(2-*p*-Tolylimidazo [1,2-*a*] pyridin-6-yl)-benzyloxy]phenyl}ethanon
6-[3-(4-Trifluormethylphenoxymethyl)phenyl]-2-*p-*tolylimidazo[1,2-*a*]pyridin
{3-[2-(3-Fluor-4-methylphenyl)imidazo[1,2-*a*] - pyridin-6-yl]phenyl}methanol
2-{3-[2-(4-Chlorphenyl)imidazo[1,2-*a*]pyridin-6-yl]phenyl}propan-2-ol
{2-[2-(4-Chlorphenyl)imidazo[1,2-*a*]pyridin-6-yl]-phenyl}methanol
{2-[2-(4-Chlorphenyl)imidazo[1,2-*a*]pyridin-6-yl]-phenyl}methanol-hydrochlorid (1:1)
racemischem 1-{3-[2-(4-Chlorphenyl)imidazo[1,2-*a*]-pyridin-6-yl]phenyl}ethanol
{3-[2-(2,4-Dichlorphenyl)imidazo[1,2-*a*]pyridin-6-yl]phenyl}methanol
{3-[2-(2,4-Difluorphenyl)imidazo[1,2-*a*]pyridin-6-yl]phenyl}methanol
{3-[2-(3,4-Difluorphenyl)imidazo[1,2-*a*]pyridin-6-yl]phenyl}methanol
{3-[2-(3,4-Difluorphenyl)imidazo[1,2-*a*]pyridin-6-yl]phenyl}methanol-hydrochlorid (1:1)
{3-[2-(2-Chlorphenyl)imidazo[1,2-*a*]pyridin-6-yl]-phenyl}methanol
{3-[2-(4-Trifluormethylphenyl)imidazo[1,2-*a*]-pyridin-6-yl]phenyl}methanol
{3-[2-(4-(Difluormethyl)phenyl)imidazo[1,2-*a*]-pyridin-6-yl]phenyl}methanol
racemischem 1-{2-[2-(4-Chlorphenyl)imidazo[1,2-*a*]-pyridin-6-yl]phenyl}ethanol
2-(4-Chlorphenyl)-6-(2-methoxymethylphenyl)-imidazo[1,2-*a*]pyridin
2-(4-Chlorphenyl)-6-(2-methoxymethylphenyl)-imidazo[1,2-*a*]pyridin-hydrochlorid (1:1)
2-(4-Chlorphenyl)-6-(4-methoxymethylphenyl)-imidazo[1,2-*a*]pyridin
racemischem 1-{3-[2-(4-Chlorphenyl)imidazo[1,2-*a*]-pyridin-6-yl]phenyl}propan-1-ol
racemischem 1-{3-[2-(4-Chlorphenyl)imidazo[1,2-*a*] - pyridin-6-yl]phenyl}pentan-1-ol
racemischem 1-{3-[2-(4-Chlorphenyl)imidazo[1,2-*a*] - pyridin-6-yl]phenyl}heptan-1-ol
racemischem 1-{3-[2-(4-Chlorphenyl)imidazo[1,2-*a*]-pyridin-6-yl]phenyl}-3-methylbutan-1-ol
racemischem {3-[2-(4-Chlorphenyl)imidazo[1,2-*a*]-pyridin-6-yl]phenyl}cyclopentylmethanol
racemischem {3-[2-(4-Chlorphenyl)imidazo[1,2-*a*]-pyridin-6-yl]phenyl}phenylmethanol
rechtsdrehendem Enantiomer von 1-{3-[2-(4-Chlorphenyl)imidazo[1,2-*a*]pyridin-6-yl]phenyl}ethanol
linksdrehendem Enantiomer von 1-{3-[2-(4-Chlorphenyl)imidazo[1,2-*a*]pyridin-6-yl]phenyl}ethanol
2-(4-Chlorphenyl)-6-(3-methoxymethylphenyl)-imidazo[1,2-*a*]pyridin
racemischem 1-{4-[2-(4-Chlorphenyl)imidazo[1,2-*a*]-pyridin-6-yl]phenyl}ethanol
racemischem 1-{3-[2-(2,4-Difluorphenyl)imidazo-[1,2-*a*]pyridin-6-yl]phenyl}ethanol
racemischem 1-{3-[2-(4-Fluorphenyl)imidazo[1,2-*a*]-pyridin-6-yl]phenyl}ethanol
racemischem 1-{3-[2-(3,4-Difluorphenyl)imidazo-[1,2-*a*]pyridin-6-yl]phenyl}ethanol
racemischem 1-{3-[2-(2-Chlorphenyl)imidazo[1,2-*a*] - pyridin-6-yl]phenyl}ethanol
racemischem 1-{3-[2-(3-Fluorphenyl)imidazo[1,2-*a*]-pyridin-6-yl]phenyl}ethanol
racemischem 1-{3-[2-(3-Chlorphenyl)imidazo[1,2-*a*]-pyridin-6-yl]phenyl}ethanol
4-[6-(3-Hydroxymethylphenyl)imidazo[1,2-*a*]pyridin-2-yl]phenol,
in Basen- oder Säureadditionssalzform.

9. Medikament, **dadurch gekennzeichnet, daß** es eine Verbindung der Formel (I) nach einem der Ansprüche 1 bis 8 oder ein Additionssalz dieser Verbindung mit einer pharmazeutisch unbedenklichen Säure enthält.

10. Pharmazeutische Zusammensetzung, **dadurch gekennzeichnet, daß** sie eine Verbindung der Formel (I) nach einem der Ansprüche 1 bis 8 oder ein pharmazeutisch unbedenkliches Salz dieser Verbindung sowie mindestens einen pharmazeutisch unbedenklichen Hilfsstoff enthält.

11. Verwendung einer Verbindung der Formel (I) nach einem der Ansprüche 1 bis 8 zur Herstellung eines Medikaments zur Behandlung und Prävention von neurodegenerativen Erkrankungen.

12. Verwendung einer Verbindung der Formel (I) nach einem der Ansprüche 1 bis 8 zur Herstellung eines Medikaments zur Behandlung und Prävention von Hirntraumen und Epilepsie.

13. Verwendung einer Verbindung der Formel (I) nach einem der Ansprüche 1 bis 8 zur Herstellung eines Medikaments zur Behandlung und Prävention von psychiatrischen Erkrankungen.

14. Verwendung einer Verbindung der Formel (I) nach einem der Ansprüche 1 bis 8 zur Herstellung eines Medikaments zur Behandlung und Prävention von entzündlichen Erkrankungen.

15. Verwendung einer Verbindung der Formel (I) nach einem der Ansprüche 1 bis 8 zur Herstellung eines Medikaments zur Behandlung und Prävention von Osteoporose und Krebserkrankungen.

16. Verwendung einer Verbindung der Formel (I) nach einem der Ansprüche 1 bis 8 zur Herstellung eines Medikaments zur Behandlung in Kombination mit Stammzellenverpflanzungen und/oder -transplantationen.

17. Verwendung einer Verbindung der Formel (I) nach einem der Ansprüche 1 bis 8 zur Herstellung eines Medikaments zur Behandlung und Prävention von Parkinson-Krankheit, Alzheimer-Krankheit, Tauopathien und multipler Sklerose.

18. Verwendung einer Verbindung der Formel (I) nach einem der Ansprüche 1 bis 8 zur Herstellung eines Medikaments zur Behandlung und Prävention von Schizophrenie, Depression, Substanzabhängigkeit und Aufmerksamkeitsdefizit-Hyperaktivitätssyndrom.

19. Verfahren zum Erhalt der Verbindungen der Formel (I) nach Anspruch 1, **dadurch gekennzeichnet, daß** man sie durch eine metallkatalysierte Kupplungsreaktion zwischen einem 2-Arylimidazopyridin der allgemeinen Formel (II) 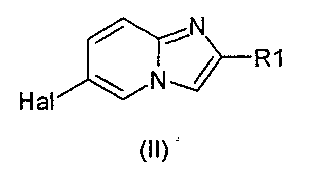 worin Hal für ein Halogenatom steht und R₁ die in Anspruch 1 angegebene Bedeutung besitzt,
und einem Derivat der allgemeinen Formel (III) 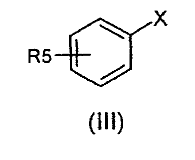 worin X für ein Bor- oder Zinnderivat steht und R₅
für die Gruppe 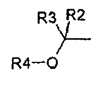 steht, erhält.

20. Verfahren zum Erhalt der Verbindungen der Formel (I) nach Anspruch 1, **dadurch gekennzeichnet, daß** man sie durch eine metallkatalysierte Kupplungsreaktion zwischen einem 2-Arylimidazopyridin der allgemeinen Formel (II) 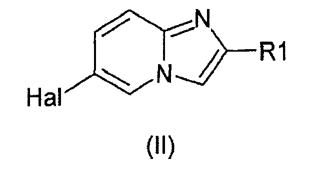 worin R₁ die in Anspruch 1 angegebene Bedeutung besitzt und Hal für ein Halogenatom steht,
und einem Derivat der allgemeinen Formel (III) 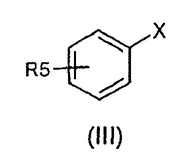 worin X für ein Bor- oder Zinnderivat steht und R₅ für ein Carbonylderivat R₂COR₃, worin R₂ und R₃ die in Anspruch 1 angegebene Bedeutung besitzen, steht,
zu Verbindungen der allgemeinen Formel (IV) 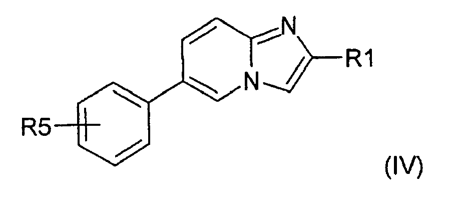 worin R₁ die in Anspruch 1 angegebene Bedeutung besitzt und R₅ für ein Carbonylderivat R₂COR₃, worin R₂ und R₃ die in Anspruch 1 angegebene Bedeutung besitzen, steht,
und nachfolgende Umwandlung der Verbindungen der allgemeinen Formel (IV) in Verbindungen der allgemeinen Formel (I) durch Einwirkung eines metallorganischen Derivats oder durch Reduktion der Carbonylgruppe mit einem Metallhydrid erhält.
